# EUROPÄISCHE PATENTSCHRIFT

(11) **EP 2 781 496 B1**
(45) Veröffentlichungstag und Bekanntmachung des Hinweises auf die Patenterteilung: **21.09.2016**
(21) Anmeldenummer: 14160170.8
(22) Anmeldetag: 17.03.2014
(51) Int. Cl.: C07C 4/22, C08F 2/40

(54) **Verfahren und Zusammensetzung zur Inhibierung der Polymerisation von Cyclopentadienverbindungen**
Method and composition for inhibiting the polymerisation of cyclopentadiene compounds
Procédé et composition permettant d'inhiber la polymérisation de composés de cyclopentadiène

(30) Priorität: 20.03.2013 DE 102013204950
(43) Veröffentlichungstag der Anmeldung: 24.09.2014
(73) Patentinhaber: Evonik Degussa GmbH, 45128 Essen (DE)
(72) Erfinder: Rinker, Stefanie, 46569 Hünxe (DE); Bressel, Bettina, 45770 Marl (DE); Neumann, Manfred, 45770 Marl (DE); Nissen, Felix, 48301 Nottuln (DE); Erpeldinger, Oliver, 42489 Wülfrath (DE); James, Phillip R., Tenby, SA70 7LE (GB); Watkins, Peter, Wokingham, West Berkshire, RG40 2 EJ (GB)

(56) Entgegenhaltungen:
- EP-A1- 2 055 691
- JP-A- 2011 256 142

## Beschreibung

Diese Erfindung betrifft eine Zusammensetzung umfassend Chinonmethide, die geeignet ist, Cyclopentadienverbindungen wie zum Beispiel Cyclopentadien und Dicyclopentadien zu stabilisieren. Sie betrifft ein Verfahren zur Inhibierung der Polymerisation von Cyclopentadienverbindungen. Die Anwendung kann in sämtlichen Prozessströmen erfolgen, die Cyclopentadienverbindungen enthalten.

### Hintergrund der Erfindung

Cyclopentadien (= CPD) ist ein sehr reaktives Molekül, das bereits in der Kälte durch Diels-Alder-Reaktion zu Dicyclopentadien (= DCPD) dimerisiert. Daher ist die dimere Form auch die, die kommerziell verfügbar ist. Durch eine Retro-Diels-Alder-Reaktion, die bei hohen Temperaturen abläuft, kann das Monomer wieder freigesetzt werden. Die reversible Dimerisierung von CPD zu DCPD lässt sich somit wie folgend in Reaktionsgleichung <1 > dargestellt beschreiben (Hinreaktion von CPD zu DCPD ist die Dimerisierung, die bevorzugt bei Kälte abläuft; Rückreaktion von DCPD zu CPD ist die Spaltung, die bevorzugt in der Hitze, d. h. T > 155 °C, abläuft):

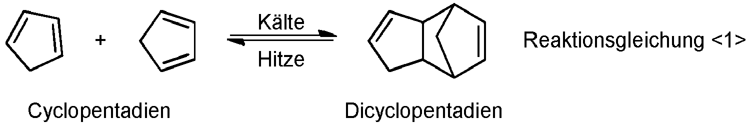

Allerdings neigt sowohl CDP als auch DCDP zur Polymerisation. Diese kann durch verschiedene Mechanismen erklärt werden. Unter anderem kann es einerseits zu weiteren Diels-Alder-Reaktionen kommen, andererseits finden auch radikalische Polymerisationen statt. Auch kann man sich gemischte Polymerisationsmechanismen vorstellen. Die Diels-Alder-Polymerisation ist in Reaktionsgleichung <2> gezeigt.

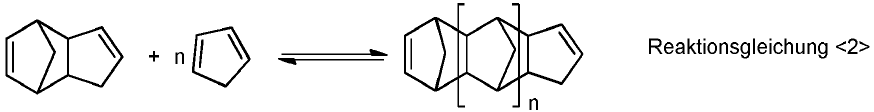

Diese hohe Polymerisationsneigung des CPD und seiner Verbindungen kann sowohl bei der gezielten Herstellung des Cyclopentadien-Monomers als auch in sämtlichen (Di-)Cyclopentadien-haltigen Prozessströmen zu vielfältigen Problemen führen.

So liegen Cyclopentadienverbindungen wie Cyclopentadien und Dicyclopentadien in manchen Prozessströmen, wie z.B. im Pyrolysebenzin, als Nebenkomponenten vor, und können mit sich selbst oder anderen vinyl-haltigen Monomeren Polymerisationsreaktionen eingehen. Diese unerwünschten Polymerisationsreaktionen treten vor allem bei hohen Temperaturen auf und können zu Ablagerungen in den Anlagen führen. Dies hat zur Folge, dass der Wärmeübertrag vermindert und somit die Produktivität verringert wird. Führen die Ablagerungen zu Verstopfungen, so müssen außerplanmäßige Reinigungen der Anlage durchgeführt werden, was zu Unterbrechungen in der Produktion führt. Jeder Ausfall bringt Kosten durch Reparatur und Reinigung, aber vor allem auch durch den Produktionsausfall selbst mit sich. Daher wird stets versucht, solche Ausfälle zu vermeiden.

Nicht nur in Prozessströmen, die Cyclopentadienverbindungen als Nebenkomponenten enthalten, sondern auch und besonders bei der Herstellung von Cyclopentadien selbst, treten die beschriebenen Probleme durch unerwünschte Polymerisation auf. Wie bereits angemerkt, wird Cyclopentadien industriell durch Spaltung ("Cracken") von Dicyclopentadien (unter Hitze ablaufende Rückreaktion in der obigen Reaktionsgleichung <1>) gewonnen. Das Cracken von Dicyclopentadien kann laut Stand der Technik sowohl in der Flüssigphase als auch in der Gasphase erfolgen (Z. Cai, B. Shen, W. Liu, Z. Xin, H. Ling, Energy & Fuels 2009, 23, 4077 - 4081). Besonders für die in der flüssigen Phase durchgeführte Variante des Crackens besteht das Problem der Bildung von Oligomerablagerungen. Auch hierbei kann es zu verminderter Wärmeübertragung, verringerter Produktivität und im Extremfall zur Verstopfung von Anlagenteilen kommen, so dass Abschaltungen und Reinigungen notwendig sind.
Für das Cracken des Dicyclopentadiens zum Cyclopentadien sind Temperaturen von mindestens 155 °C nötig. Bei diesen Temperaturen entstehen sehr schnell Oligo- und Polymere des Cyclopentadien, die die Viskosität erhöhen und dadurch die Rührung erschweren, sich ablagern und einen effektiven Wärmeübergang verhindern, so dass die Reaktion schwerer durchzuführen ist und es zu Ausbeuteverlusten kommt.

Im Stand der Technik werden verschiedene Strategien beschrieben, um der Bildung von Oligomeren beim Cracken des Dicyclopentadiens entgegenzuwirken. Die verbreitetste Möglichkeit ist die Zugabe eines inerten Lösungsmittels als Sumpfverdünner. Dabei werden vor allem langkettige Kohlenwasserstoffe eingesetzt. Das Lösungsmittel verhindert die Polymerisation zwar nicht, verlangsamt sie aber, da es die Konzentration der reaktiven Komponente verringert. Der Vorteil eines solchen Lösungsmitteleinsatzes besteht darin, dass sich die gebildeten Oligomere in dem Lösungsmittel lösen und sich somit nicht ablagern, wodurch die Reaktionsmischung bearbeitbar bleibt. Zudem kann durch Einsatz eines Lösungsmittels die Zeit der thermischen Belastung herabgesetzt werden. Dieser Vorteil wird beispielsweise von Ammannati et al. in WO2010/020549 beschrieben. Hier wird Diphenylether als inertes Lösungsmittel eingesetzt. Robota (DE1951320; GB1261565) beschreibt ein Crackverfahren, in welchem ein paraffinbasisches Kohlenwasserstofföl als Lösungsmittel eingesetzt wird.

Andererseits bringt der Einsatz eines inerten Lösungsmittels den Nachteil mit sich, dass ein Teil des Reaktorvolumens bereits durch das Lösungsmittel besetzt ist, so dass die Volumen-Zeit-Ausbeute durch den Lösungsmittelzusatz verringert ist.

Eine weitere im Stand der Technik beschriebene Möglichkeit, unerwünschte Polymerisationen zu verhindern, bietet der Einsatz von Polymerisationsinhibitoren. Diese Möglichkeit wird vor allem zur Vermeidung der Polymerisation in Prozessströmen genutzt, die verschiedene, zum Teil vinylische, Monomere, aber auch DCPD und CPD enthalten. Hierbei sind folgende Inhibitorklassen beschrieben: Nitroxide wie TEMPO-Derivate, Phenyldiamine, Hydroxylamine wie Diethylhydroxylamin (abgekürzt als "DEHA"), Nitroaromaten wie 4,6-Dinitro-2-sec-Butylphenol (abgekürzt als "DNBP"), Diphenole wie Hydrochinon (abgekürzt als "HQ") und p-*tert*-Butylcatechol (abgekürzt als "TBC").

Buccolini et al. (WO2001/047844) beschreiben den Einsatz einer Kombination von Nitroxidverbindungen, insbesondere TEMPO-Derivaten, und aromatischen Aminen, insbesondere Diphenylaminen und Phenylendiaminen, zur Inhibierung von Polymerisationsreaktionen in Kohlenwasserstoffströmen. Diese Kohlenwasserstoffströme enthalten Butadien und Styrol, können aber auch Cyclopentadien enthalten.

Kazuo et al. (JP62167733A & JP62167734A) beschreiben die Beimischung von Hydroxylaminen bzw. TEMPO-Derivaten zu einer Reaktionsmischung, beispielsweise aus Cyclopentadien und Dicyclopentadien mit 1,3-Butadien, um Polymerisationsnebenreaktionen zu unterbinden.

Ammannati et al. (WO2010/020549) beschreiben ein Verfahren zur Herstellung von Ethylidennorbornen, in welchem in einem ersten Schritt Dicyclopentadien zu Cyclopentadien umgesetzt wird. Es können dabei verschiedene inerte Lösungsmittel (z. B. Diphenylether, Diphenylmethan, Dekalin oder einer Mischung aus Di- und Triarylethern) eingesetzt werden. Zusätzlich werden Polymerisationsinhibitoren wie beispielsweise 4-Oxo-2,2,6,6-tetramethylpiperidin-*N*-oxid (abgekürzt als "4-Oxo-TEMPO") sowie *tert-*Butylhydrochinon eingesetzt.
Die JP 2011-256142 A beschreibt mit Phenyl-*N'*-p-phenylendiaminderivaten eine andere Substanzklasse, welche zur Inhibierung der Polymerisation bestimmter Monomere, darunter auch Cyclopentadien, eingesetzt werden kann.
Cai *et al.* (Z. Cai, B. Shen, W. Liu, Z. Xin, H. Ling, Energy & Fuels 2009, 23, 4077 - 4081) beschreiben o-Nitrophenol, TBC und DEHA als mögliche Inhibitoren. Als besonders vorteilhaft wird dabei eine 3 zu 1 Mischung (Gewichtsanteile) von TBC und DEHA genannt.

Cheng et al. (CN101798255) beschreiben Polymerisationsinhibitoren, die beim Abtrennen von Diolefinen aus der C5-Fraktion in einem Cracking-Prozess zum Einsatz kommen können, speziell bei der Extraktion von 1,3-Pentadien. Als mögliche Polymerisationsinhibitoren werden Natriumnitrit, TBC, DEHA und o-Nitrophenol erwähnt.

Ge et al. (CN101104573) beschreiben ein Verfahren zur Trennung von Isopren und Cyclopentadien, in welchem Polymerisationsinhibitoren eingesetzt werden. Als Inhibitoren können eine oder mehrere der Substanzen ausgewählt aus o-Nitrophenol, TBC, DEHA und Dihydroxydihydrozimtsäure eingesetzt werden.

Chen et al. (CN102060649) beschreiben ein Verfahren zur Herstellung von Cyclopentadien, in welchem HQ, 2,6-Dinitrokresol und/oder TBC oder Phenothiazin (abgekürzt als "PTZ") zum Einsatz kommen.

Hu et al. (CN1253130) beschreiben ein Verfahren zur Abtrennung von Diolefinen aus einem C5-Strom, wobei DEHA, TBC oder o-Nitrophenol als Stabilisatoren benutzt werden können.

Lartigue-Peyrou et al. (WO1999/015603) beschreiben Mischungen aus Catecholderivaten und aromatischen Ethern, die zur Stabilisierung ungesättigter Verbindungen, darunter auch Cyclopentadien und Dicyclopentadien, genutzt werden können.

Cocu 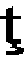 a *et al.* (RO93489) beschreiben Mischungen aus sec-Butylphenolen und Phenylendiaminen bzw. *tert*-Butylcatecholen, die die Polymerisation von Olefinen und Diolefinen verhindern können.
Bei Experimenten, bei denen reines Cyclopentadien eingesetzt wird, zeigt sich allerdings überraschend, dass die meisten im Stand der Technik beschriebenen Inhibitoren, die u.a. auch für die Anwendung in Anwesenheit von Cyclopentadien eingesetzt werden, für die Vermeidung der Polymerisation des Cyclopentadiens nur schlechte oder gar keine Wirksamkeit haben (siehe Vergleichsbeispiele 1 bis 6).

Dies betrifft beispielsweise die in den WO2010/020549, WO2001/047844 und JP62167734 beschriebenen Substanzen, die als Inhibitoren bezüglich der Polymerisationsreaktionen von Monomeren wie Butadien oder Styrol (siehe Vergleichsbeispiele 13 bis 16) wirksam sind. Wie in den Vergleichsbeispielen 1 bis 6 dargelegt, versagen diese Substanzen jedoch, wenn sie zur Inhibierung der Polymerisation von Cyclopentadien eingesetzt werden. So wurde festgestellt, dass z. B. DNBP in Bezug auf die Polymerisation von Cyclopentadien keine Wirkung aufweist. Auch die TEMPO-Derivate haben nur eine schwache Wirksamkeit.

Es wurde demnach überraschend festgestellt, dass die im Stand der Technik beschriebenen Inhibitoren zur Vermeidung der Polymerisation in CPD oder DCPD-haltigen Prozessströmen wenig geeignet sind und hauptsächlich oder sogar ausschließlich als Inhibitoren zur Vermeidung von radikalischen Polymerisationen, die bei vinylhaltigen Monomeren beobachtet werden, wirksam sind. Die Tatsache, dass bestimmte Substanzen allgemein zur Stabilisierung von olefinisch ungesättigten Monomeren beschrieben wurden, lässt demnach keinen Rückschluss über ihre Eignung als Inhibitor der Polymerisierung von Cyclopentadien, Dicyclopentadien oder anderen Cyclopentadienverbindungen zu.

Die Aufgabe der vorliegenden Erfindung war deshalb, einen Polymerisationsinhibitor zur Verfügung zu stellen, der eine gute Aktivität gegen unerwünschte Polymerisationen von Dicyclopentadien und Cyclopentadien zeigt. Der Inhibitor sollte zudem bei hohen bis sehr hohen Temperaturen wirken und gegenüber den im Stand der Technik beschriebenen Inhibitoren eine verbesserte Wirkung aufweisen.

Es wurde nun völlig überraschend gefunden, dass diese Aufgabe durch den Einsatz bestimmter Chinonmethide [im Folgenden "Verbindung der Struktur (I)"] gelöst werden kann. Diese weisen hervorragende Inhibitionseigenschaften bezüglich der Polymerisation von CPD und DCPD auf, was an sich schon überraschend ist, da sie bisher lediglich zur Vermeidung der Polymerisation von vinylischen Monomeren (EP2055691A1; EP0737659A1; EP0737660A1, WO2012/173909) beschrieben sind. Beispielsweise beschreibt die EP2055691A1 lediglich den Einsatz dieser Chinonmethide als Inhibitoren der Polymerisation von olefinisch ungesättigten Monomeren wie zum Beispiel Ethen, Propen, Butadien, Vinylacetat, vor allem aber von Styrol. Weiterhin ist überraschend, dass die erfindungsgemäßen Chinonmethide in ihrer Inhibitionswirkung die im Stand der Technik beschriebenen Inhibitoren der Polymerisierung von CPD und DCPD übertreffen. Die erfindungsgemäßen Chinonmethide können unverdünnt eingesetzt werden, aber auch in verdünnter Form. Die Cyclopentadienverbindung kann als Reinstoff, aber auch in einem Prozessstrom vorliegen.

### Kurzbeschreibung der Erfindung

Die Erfindung betrifft eine Zusammensetzung (AB) umfassend (A) und (B), ein Verfahren zur Inhibierung der Polymerisation von (B), was dadurch gekennzeichnet ist, dass (A) und (B) in Kontakt gebracht werden, sowie die Verwendung von (A) zur Inhibierung der Polymerisation von (B),
wobei
(A) mindestens eine Verbindung der Struktur (I) 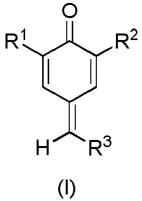 mit
   R¹ und R² sind unabhängig voneinander Wasserstoff, Alkylgruppe mit 1 bis 18 Kohlenstoffatomen, Cycloalkylgruppe mit 3 bis 15 Kohlenstoffatomen, Arylgruppe mit 6 bis 15 Kohlenstoffatomen oder Phenylalkylgruppe mit 7 bis 15 Kohlenstoffatomen;
   R³ = -CN, -COOH, -COOR⁴, -COR⁵, -OCOR⁶, -CONR⁷R⁸, -PO(OR⁹)₂, -O-R¹⁰ ,-S-R¹¹, -R¹², -C≡C-R¹³ oder Halogen;
      mit
      R⁴, R⁵, R⁶ = Alkylgruppe mit 1 bis 18 Kohlenstoffatomen, Cycloalkylgruppe mit 3 bis 12 Kohlenstoffatomen, Arylgruppe mit 6 bis 10 Kohlenstoffatomen;
      R⁷ und R⁸ sind unabhängig voneinander
         Wasserstoff;
         Alkylgruppe mit 1 bis 15 Kohlenstoffatomen, die substituiert sein kann mit mindestens einem Substituenten ausgewählt aus der Gruppe bestehend aus Alkylaminogruppe mit 1 bis 4 Kohlenstoffatomen, Dialkylaminogruppe mit 2 bis 8 Kohlenstoffatomen, Hydroxygruppe;
         Phenylalkylgruppe mit 7 bis 15 Kohlenstoffatomen, die substituiert sein kann mit mindestens einem Substituenten ausgewählt aus der Gruppe bestehend aus Hydroxygruppe, Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen;
         Arylgruppe mit 6 bis 10 Kohlenstoffatomen, die substituiert sein kann mit mindestens einem Substituenten ausgewählt aus der Gruppe bestehend aus Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen, Hydroxygruppe;
         oder NR⁷R⁸ ist Morpholino, Piperidino oder Pyrrolidino;
      R⁹, R¹⁰, R¹¹ =
         Wasserstoff;
         Alkylgruppe mit 1 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Cycloalkylgruppe mit 3 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Phenylalkylgruppe mit 7 bis 15 Kohlenstoffatomen die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen; oder
         Arylgruppe mit 6 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
      R¹² = 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen; wobei die Reste 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
      R¹³ = Wasserstoff, Alkylgruppe mit 1 bis 12 Kohlenstoffatomen, Arylgruppe mit 6 bis 10 Kohlenstoffatomen, wobei die Arylgruppe mit 6 bis 10 Kohlenstoffatomen substituiert sein kann mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
      wobei die Substituenten vom Typ R¹, R², R³ gleich oder unterschiedlich sind,
      ist,
      und
(B) mindestens eine Cyclopentadienverbindung
   ist.

### Abbildungen

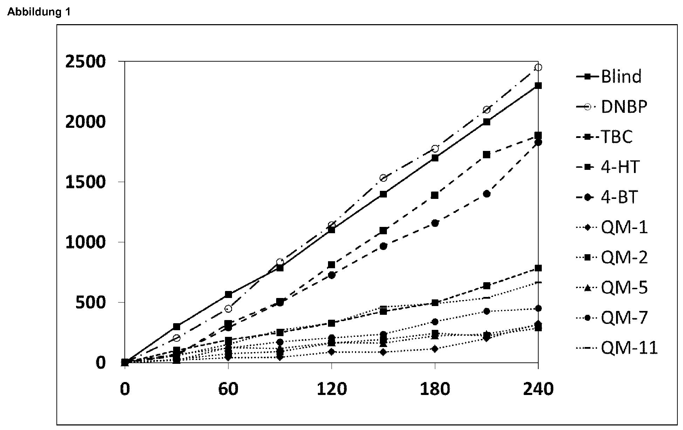

Abbildung 1 zeigt die Ergebnisse, die in den Beispielen 1 bis 11 bei der Austestung bestimmter Verbindungen des Standes der Technik im Vergleich zu den erfindungsgemäßen Verbindungen bezüglich ihrer Wirksamkeit zur Stabilisierung von reinem (Di-)Cyclopentadien gegen Polymerisation bei 170 °C Ölbadtemperatur erhalten wurden. Die x-Achse gibt die Zeit in Minuten an, die y-Achse die mit ELS gemessene Peakfläche. Abkürzungen: 4-Hydroxy-TEMPO (4-HT), 4-Butoxy-TEMPO (4-BT), *tert*-Butyl-Catechol (TBC) bzw. Dinitro-sec-Butylphenol (DNBP). Die Struktur von **QM-1** ist (V), die Struktur von **QM-2** ist (VI), die Struktur von **QM-5** ist (X), die Struktur von **QM-7** ist (XII) und die Struktur von **QM-11** ist (XVI).

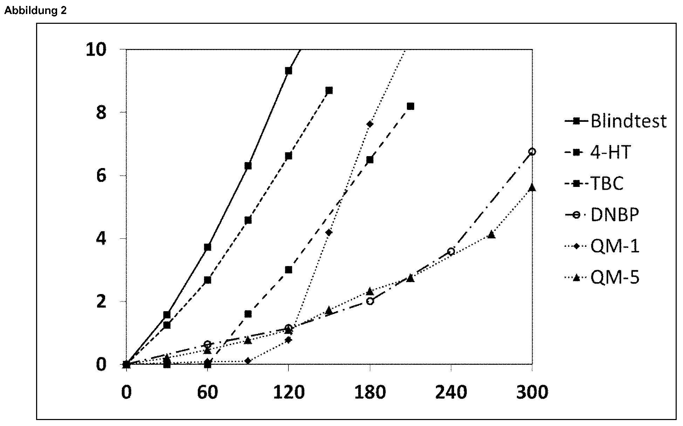

Abbildung 2 zeigt einige Ergebnisse, die in den Beispielen 12 bis 21 bei der Austestung bestimmter Verbindungen des Standes der Technik im Vergleich zu den erfindungsgemäßen Verbindungen bezüglich ihrer Wirksamkeit zur Stabilisierung von Styrol gegen Polymerisation bei 110 °C erhalten wurden. Die x-Achse gibt die Zeit in Minuten an, die y-Achse den Polymergehalt in %. Weitere Ergebnisse sind in Tabelle 2 zu finden. Abkürzungen: 4-Hydroxy-TEMPO (4-HT), *tert*-Butyl-Catechol (TBC) bzw. Dinitro-sec-Butylphenol (DNBP). Die Struktur von **QM-1** ist (V), die Struktur von **QM-5** ist (X).

Die genauen Versuchsbeschreibungen sind im Abschnitt "Beispielteil" zu finden.

### Detaillierte Beschreibung der Erfindung

### Allgemeine Begriffe

Der Begriff "Cyclopentadienverbindung" im Sinne der vorliegenden Erfindung umfasst eine Verbindung, die ausgewählt ist aus der Gruppe bestehend aus Cyclopentadien, Dicyclopentadien, alkyliertes Cyclopentadien und alkyliertes Dicyclopentadien; bevorzugt ausgewählt ist aus der Gruppe bestehend aus Cyclopentadien und Dicyclopentadien.

Cyclopentadien (CPD) hat die Struktur (II).

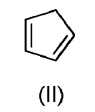

Dicyclopentadien (DCPD) hat die Struktur (III) und besitzt zwei isomere Formen, *endo-*Dicyclopentadien (endo-DCPD) und *exo*-Dicyclopentadien (exo-DCPD).

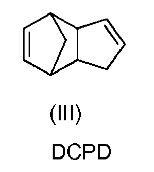

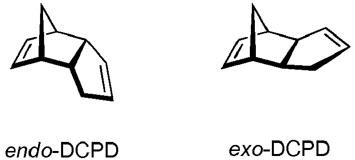

Der Begriff "(Di-)Cyclopentadien" bezeichnet im Sinne der vorliegenden Erfindung Mischungen aus CPD und DCPD.

Der Ausdruck "Polymerisation von (B)" umfasst jede Polymerisation, in der (B) beteiligt ist, bevorzugt eine Oligomerisierung/Polymerisierung mit sich selbst oder vinylischen Strukturen.

Der Begriff "alkyliertes Cyclopentadien" im Sinne der vorliegenden Erfindung umfasst Verbindungen der Struktur (IV). 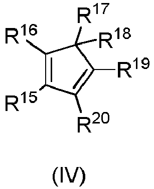 wobei in der Verbindung der Struktur (IV) mindestens einer der Reste R¹⁵, R¹⁶, R¹⁷, R¹⁸, R¹⁹, R²⁰ = Alkylgruppe mit 1 bis 18 Kohlenstoffatomen und die übrigen Reste Wasserstoff sind. In einer bevorzugten Ausführungsform handelt es sich bei dem "alkylierten Cyclopentadien" um Monoalkylcyclopentadien oder Dialkylcyclopentadien, besonders bevorzugt um Monoalkylcyclopentadien.

Der Begriff "Monoalkylcyclopentadien" im Sinne der vorliegenden Erfindung umfasst die Verbindungen der Struktur (IV), wobei genau einer der Reste R¹⁵, R¹⁶, R¹⁷, R¹⁸, R¹⁹, R²⁰ = Alkylgruppe mit 1 bis 18 Kohlenstoffatomen, bevorzugt Alkylgruppe mit 1 bis 6 Kohlenstoffatomen, bevorzugter eine Methylgruppe oder eine Ethylgruppe, am bevorzugtesten eine Methylgruppe ist, und die übrigen Reste jeweils Wasserstoff sind.

Der Begriff "Dialkylcyclopentadien" im Sinne der vorliegenden Erfindung umfasst die Verbindungen der Struktur (IV), wobei genau zwei der Reste R¹⁵, R¹⁶, R¹⁷, R¹⁸, R¹⁹, R²⁰ unabhängig voneinander jeweils eine Alkylgruppe mit 1 bis 18 Kohlenstoffatomen, bevorzugt eine Alkylgruppe mit 1 bis 6 Kohlenstoffatomen, bevorzugter eine Methylgruppe oder eine Ethylgruppe, am bevorzugtesten eine Methylgruppe sind, und die übrigen Reste jeweils Wasserstoff sind.

Der Begriff "alkyliertes Dicyclopentadien" im Sinne der vorliegenden Erfindung umfasst alle Moleküle der Struktur (III), in welcher mindestens ein Wasserstoffrest durch eine Alkylgruppe mit 1 bis 18 Kohlenstoffatomen, bevorzugt durch eine Alkylgruppe mit 1 bis 6 Kohlenstoffatomen, besonders bevorzugt durch eine Methylgruppe oder Ethylgruppe substituiert ist.

Der Begriff "alkyliertes Dicyclopentadien" im Sinne der vorliegenden Erfindung umfasst in einer ganz besonders bevorzugten Ausführungsform alle Moleküle der Struktur (III), in welchen genau ein Wasserstoffrest, genau zwei Wasserstoffreste, genau drei Wasserstoffreste oder genau vier Wasserstoffreste durch eine Alkylgruppe mit 1 bis 6 Kohlenstoffatomen, besonders bevorzugt durch eine Methylgruppe oder Ethylgruppe substituiert ist.

Eine Alkylgruppe mit 1 bis 18 Kohlenstoffatomen weist im Sinne der vorliegenden Erfindung zwischen 1 und 18 gesättigte Kohlenstoffatome auf und kann dabei linear oder verzweigt sein und kann insbesondere ausgewählt sein aus Methyl, Ethyl, *n*-Propyl, *iso*-Propyl, *n*-Butyl, sec-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutly, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl, 1-Ethyl-2-methylpropyl, Heptyl, Octyl, Nonyl, Decyl, Undecyl, Dodecyl, Tridecyl, Tetradecyl, Pentadecyl, Hexadecyl, Heptadecyl, Octadecyl. Eine Alkylgruppe mit 1 bis 12 Kohlenstoffatomen weist im Sinne der vorliegenden Erfindung zwischen 1 und 12 gesättigte Kohlenstoffatome auf und kann dabei linear oder verzweigt sein und kann insbesondere ausgewählt sein aus Methyl, Ethyl, *n*-Propyl, *iso*-Propyl, *n*-Butyl, sec-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutly, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl, 1-Ethyl-2-methylpropyl, Heptyl, Octyl, Nonyl, Decyl, Undecyl, Dodecyl.

Eine Alkylgruppe mit 1 bis 6 Kohlenstoffatomen weist im Sinne der vorliegenden Erfindung zwischen 1 und 6 gesättigte Kohlenstoffatome auf und kann dabei linear oder verzweigt sein und kann insbesondere ausgewählt sein aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl, 1-Ethyl-2-methylpropyl.

Eine Alkylgruppe mit 1 bis 4 Kohlenstoffatomen weist im Sinne der vorliegenden Erfindung zwischen 1 und 4 gesättigte Kohlenstoffatome auf kann dabei linear oder verzweigt sein und kann insbesondere ausgewählt sein aus Methyl, Ethyl, *n*-Propyl, *iso*-Propyl, *n*-Butyl, *sec*-Butyl, *iso*-Butyl, *tert*-Butyl*.*

Eine Alkylgruppe mit 1 bis 3 Kohlenstoffatomen weist im Sinne der vorliegenden Erfindung zwischen 1 und 3 gesättigte Kohlenstoffatome auf kann dabei linear oder verzweigt sein und kann insbesondere ausgewählt sein aus Methyl, Ethyl, *n*-Propyl, *iso*-Propyl.

Eine Cycloalkylgruppe mit 3 bis 15 Kohlenstoffatomen ist im Sinne der vorliegenden Erfindung insbesondere ausgewählt aus Cyclopropyl, Cyclobutyl, Cyclopropylmethyl, Cyclopentyl, Cyclobutylmethyl, Cyclohexyl, Cycloheptyl, Cyclooctyl, Cyclononyl, Cyclodecyl, Cycloundecyl, Cyclododecyl, Cyclotridecyl, Cyclotetradecyl, Cyclopentadecyl.

Eine Cycloalkylgruppe mit 3 bis 12 Kohlenstoffatomen ist im Sinne der vorliegenden Erfindung insbesondere ausgewählt aus Cyclopropyl, Cyclobutyl, Cyclopropylmethyl, Cyclopentyl, Cyclobutylmethyl, Cyclohexyl, Cycloheptyl, Cyclooctyl, Cyclononyl, Cyclodecyl, Cycloundecyl, Cyclododecyl.

Eine Arylgruppe mit 6 bis 15 Kohlenstoffatomen ist insbesondere ausgewählt aus Phenyl, 1-Naphthyl, 2-Naphthyl, 9-Anthryl, 9-Phenanthryl.

Eine Arylgruppe mit 6 bis 10 Kohlenstoffatomen ist im Sinne der vorliegenden Erfindung insbesondere ausgewählt aus Phenyl, 1-Naphthyl, 2-Naphthyl.

Eine Phenylalkylgruppe mit 7 bis 15 Kohlenstoffatomen weist im Sinne der vorliegenden Erfindung einen verzweigten oder unverzweigten Alkylrest, an den ein Phenylring geknüpft ist, auf, und ist insbesondere ausgewählt aus Benzyl, Phenylethyl, α-Methylbenzyl, 3-Phenylpropyl, Phenyl-2-methylethyl, Phenyl-1-methylethyl, α,α-Dimethylbenzyl, Butylphenyl, Hexylphenyl, Octylphenyl, Nonylphenyl, bevorzugt Benzyl.

Eine Alkylaminogruppe mit 1 bis 4 Kohlenstoffatomen bedeutet im Sinne der vorliegenden Erfindung insbesondere ein Aminorest, der eine Alkylgruppe mit 1 bis 4 Kohlenstoffatomen aufweist, und ist bevorzugt ausgewählt aus Methylamino, Ethylamino, Propylamino, Isopropylamino und Butylamino.

Eine Dialkylaminogruppe ist im Sinne der vorliegenden Erfindung insbesondere ein Aminorest, der zwei Alkylgruppen trägt und ist insbesondere eine Dialkylaminogruppe mit 2 bis 8 Kohlenstoffatomen.

Eine Dialkylaminogruppe mit 2 bis 8 Kohlenstoffatomen bedeutet im Sinne der vorliegenden Erfindung insbesondere ein Aminorest, der zwei Alkylgruppen mit 1 bis 4 Kohlenstoffatomen aufweist, wobei diese Alkylgruppen mit 1 bis 4 Kohlenstoffatomen gleich oder verschieden sein können, und ist bevorzugt ausgewählt aus Dimethylamino, Diethylamino, Dipropylamino, Dibutylamino, Methylethylamino und Methylbutylamino.

"Hochsiedende Kohlenwasserstoffschnitte" bedeutet im Sinne der vorliegenden Erfindung aliphatische oder aromatische Kohlenwasserstoffschnitte mit festgelegten Siedebereichen, insbesondere aromatische Kohlenwasserstoffe mit einem Siedepunkt (bei Normaldruck) im Bereich von 155 °C bis 300 °C, diese enthalten bevorzugt eine oder mehrere Substanzen ausgewählt aus der Gruppe bestehend aus *n*-Propylbenzol, 1-Methyl-4-ethylbenzol, 1-Methyl-3-ethylbenzol, Mesitylen, 1-Methyl-2-ethylbenzol, 1,2,4-Trimethylbenzol, 1,2,3-Trimethylbenzol, Indan, 1,3-Diethylbenzol, 1-Methyl-4-propylbenzol, 1-Methyl-3-propylbenzol, 1,2,4,5-Tetramethylbenzol, 1,2,3,5-Tetramethylbenzol und Naphthalin.

### Erfindungsgemäßes Verfahren

Der Ausdruck "erfindungsgemäßes Verfahren" ist gleichbedeutend mit "Verfahren zur Inhibierung der Polymerisation von (B)".

Die Erfindung betrifft ein Verfahren zur Inhibierung der Polymerisation von (B), was dadurch gekennzeichnet ist, dass (A) und (B) in Kontakt gebracht werden,
wobei
(A) mindestens eine Verbindung der Struktur (I) 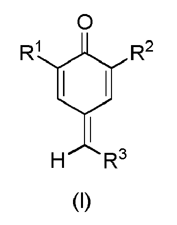 mit
   R¹ und R² sind unabhängig voneinander Wasserstoff, Alkylgruppe mit 1 bis 18 Kohlenstoffatomen, Cycloalkylgruppe mit 3 bis 15 Kohlenstoffatomen, Arylgruppe mit 6 bis 15 Kohlenstoffatomen oder Phenylalkylgruppe mit 7 bis 15 Kohlenstoffatomen;
   R³ = -CN, -COOH, -COOR⁴, -COR⁵, -OCOR⁶, -CONR⁷R⁸, -PO(OR⁹)₂, -O-R¹⁰ ,-S-R¹¹, -R¹², -C≡C-R¹³ oder Halogen;
      mit
      R⁴, R⁵, R⁶ = Alkylgruppe mit 1 bis 18 Kohlenstoffatomen, Cycloalkylgruppe mit 3 bis 12 Kohlenstoffatomen, Arylgruppe mit 6 bis 10 Kohlenstoffatomen;
      R⁷ und R⁸ sind unabhängig voneinander
         Wasserstoff;
         Alkylgruppe mit 1 bis 15 Kohlenstoffatomen, die substituiert sein kann mit mindestens einem Substituenten ausgewählt aus der Gruppe bestehend aus Alkylaminogruppe mit 1 bis 4 Kohlenstoffatomen, Dialkylaminogruppe mit 2 bis 8 Kohlenstoffatomen, Hydroxygruppe;
         Phenylalkylgruppe mit 7 bis 15 Kohlenstoffatomen, die substituiert sein kann mit mindestens einem Substituenten ausgewählt aus der Gruppe bestehend aus Hydroxygruppe, Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen;
         Arylgruppe mit 6 bis 10 Kohlenstoffatomen, die substituiert sein kann mit mindestens einem Substituenten ausgewählt aus der Gruppe bestehend aus Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen, Hydroxygruppe;
         oder NR⁷R⁸ ist Morpholino, Piperidino oder Pyrrolidino;
      R⁹, R¹⁰, R¹¹ =
         Wasserstoff;
         Alkylgruppe mit 1 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Cycloalkylgruppe mit 3 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Phenylalkylgruppe mit 7 bis 15 Kohlenstoffatomen die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen; oder
         Arylgruppe mit 6 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
      R¹² = 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen; wobei die Reste 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
      R¹³ = Wasserstoff, Alkylgruppe mit 1 bis 12 Kohlenstoffatomen, Arylgruppe mit 6 bis 10 Kohlenstoffatomen, wobei die Arylgruppe mit 6 bis 10 Kohlenstoffatomen substituiert sein kann mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
      wobei die Substituenten vom Typ R¹, R², R³ gleich oder unterschiedlich sind,
      ist,
      und
(B) mindestens eine Cyclopentadienverbindung
   ist.

In einer bevorzugten Ausführungsform des erfindungsgemäßen Verfahrens sind R¹ und R² in der Verbindung der Struktur (I) unabhängig voneinander ausgewählt aus der Gruppe bestehend aus Methyl, Ethyl, *n*-Propyl, *iso*-Propyl, *n*-Butyl, *iso*-Butyl und *tert*-Butyl; und R³ = -CN, -COOH, -COOR⁴, -COR⁵, -OCOR⁶, -CONR⁷R⁸, -PO(OR⁹)₂, -O-R¹⁰ ,-S-R¹¹, -R¹² -C≡C-R¹³ oder Halogen;
mit
R⁴, R⁵, R⁶ = Alkylgruppe mit 1 bis 8 Kohlenstoffatomen oder Phenyl;
R⁷R⁸ sind unabhängig voneinander Wasserstoff oder eine Alkylgruppe mit 1 bis 4 Kohlenstoffatomen, oder NR⁷R⁸ ist Morpholino oder Piperidino;
R⁹, R¹⁰, R¹¹ = Alkylgruppe mit 1 bis 8 Kohlenstoffatomen oder Phenyl;
R¹² = 2-Furylgruppe, 3-Furylgruppe oder Arylgruppe mit 6 bis 15 Kohlenstoffatomen, wobei die Reste 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Alkylgruppe mit 1 bis 8 Kohlenstoffatomen;
R¹³ = Wasserstoff, Alkylgruppe mit 1 bis 12 Kohlenstoffatomen, Arylgruppe mit 6 bis 10 Kohlenstoffatomen.

In einer bevorzugteren Ausführungsform des erfindungsgemäßen Verfahrens sind R¹ und R² in der Verbindung der Struktur (I) unabhängig voneinander ausgewählt aus der Gruppe bestehend aus Methyl und *tert*-Butyl; und
R³ = -CN, -COOH, -COOR⁴, -O-R¹⁰, -S-R¹¹, -R¹², -C≡C-R¹³ oder Halogen;
mit
R⁴ = Alkylgruppe mit 1 bis 4 Kohlenstoffatomen;
R¹⁰, R¹¹ = Alkylgruppe mit 1 bis 6 Kohlenstoffatomen;
R¹² = 2-Furylgruppe, 3-Furylgruppe oder Arylgruppe mit 6 bis 12 Kohlenstoffatomen, wobei die Reste 2-Furylgruppe, 3-Furylgruppe oder Arylgruppe mit 6 bis 12 Kohlenstoffatomen substituiert sein können mit mindestens einem Substituenten ausgewählt aus der Gruppe bestehend aus Hydroxygruppe, Alkylgruppe mit 1 bis 8 Kohlenstoffatomen;
R¹³ = Arylgruppe mit 6 bis 10 Kohlenstoffatomen.

In einer noch bevorzugteren Ausführungsform des erfindungsgemäßen Verfahrens sind R¹ und R² in der Verbindung der Struktur (I) *tert*-Butyl; und
R³ = -CN, -COOH, -O-R¹⁰, -S-R¹¹, -R¹² oder -C≡C-R¹³;
mit
R¹⁰ = Methyl, Ethyl, *iso*-Propyl, *n*-Propyl, *n*-Butyl, *iso*-Butyl, sec-Butyl, tert-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl, bevorzugt Methyl, Ethyl, iso-Propyl, n-Propyl, sec-Butyl, n-Butyl, n-Pentyl oder n-Hexyl;
R¹¹ = Alkylgruppe mit 1 bis 6 Kohlenstoffatomen;
R¹² = 2-Furylgruppe, 3-Furylgruppe oder Phenyl, wobei Phenyl substituiert sein kann mit mindestens einem Substituenten ausgewählt aus der Gruppe bestehend aus Hydroxygruppe, Alkylgruppe mit 1 bis 8 Kohlenstoffatomen;
R¹³ = Phenyl.

In einer ersten ganz besonders bevorzugten Ausführungsform des erfindungsgemäßen Verfahrens ist in der Verbindung der Struktur (I) R¹ und R² = *tert-*Butyl und R³ = CN, wobei die Verbindung der Struktur (I) dann die Struktur (V) aufweist (im Folgenden auch abgekürzt als **"QM-1** ") 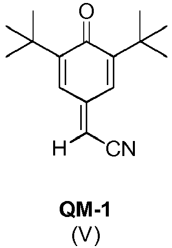 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zweiten ganz besonders bevorzugten Ausführungsform des erfindungsgemäßen Verfahrens ist in der Verbindung der Struktur (I) R¹ und R² *= tert-*Butyl und R³ = Phenyl, wobei die Verbindung der Struktur (I) dann die Struktur (VI) aufweist (im Folgenden auch abgekürzt als **"QM-2"**) 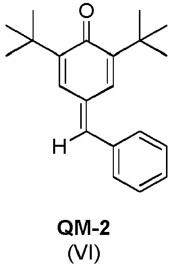 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer dritten ganz besonders bevorzugten Ausführungsform des erfindungsgemäßen Verfahrens ist in der Verbindung der Struktur (I) R¹ und R² = *tert-*Butyl und R³ = 3,5-Di-*tert-*Butyl-4-Hydroxy-Phenyl, wobei die Verbindung der Struktur (I) dann die Struktur (VII) aufweist (im Folgenden auch abgekürzt als **"QM-3")** 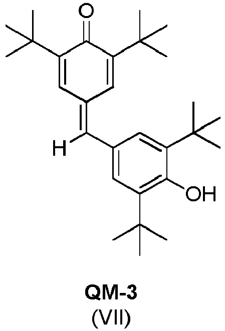 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer vierten ganz besonders bevorzugten Ausführungsform des erfindungsgemäßen Verfahrens ist in der Verbindung der Struktur (I) R¹ und R² *= tert-*Butyl und R³ = 2-Furyl, wobei die Verbindung der Struktur (I) dann die Struktur (VIII) aufweist (im Folgenden auch abgekürzt als **"QM-4")** 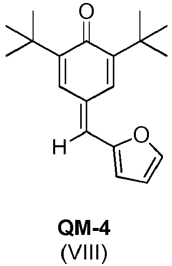 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer fünften ganz besonders bevorzugten Ausführungsform des erfindungsgemäßen Verfahrens ist in der Verbindung der Struktur (I) R¹ und R² = *tert*-Butyl und R³ = -OR¹⁰, wobei
R¹⁰ = Methyl, Ethyl, *iso*-Propyl, *n*-Propyl, *n*-Butyl, sec-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl, bevorzugt Methyl, Ethyl, *iso*-Propyl, *n*-Propyl, *n*-Butyl, *sec*-Butyl, *tert*-Butyl, *n*-Pentyl oder *n*-Hexyl, besonders bevorzugt Methyl, Ethyl, *n*-Propyl, *iso*-Propyl, *n*-Butyl, *n*-Pentyl oder *n*-Hexyl;
wobei die Verbindung der Struktur (I) dann die Struktur (IX) aufweist 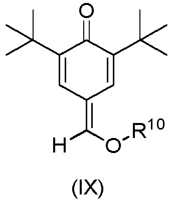 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer sechsten ganz besonders bevorzugten Ausführungsform des erfindungsgemäßen Verfahrens ist in der Verbindung der Struktur (I) R¹ und R² = *tert-*Butyl und R³ = -OCH₃, wobei die Verbindung der Struktur (I) dann die Struktur (X) aufweist (im Folgenden auch abgekürzt als **"QM-5")** 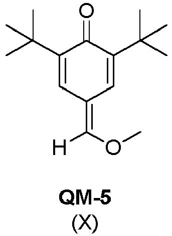 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer siebten ganz besonders bevorzugten Ausführungsform des erfindungsgemäßen Verfahrens ist in der Verbindung der Struktur (I) R¹ und R² = *tert*-Butyl und R³ = -OCH₂CH₃, wobei die Verbindung der Struktur (I) dann die Struktur (XI) aufweist (im Folgenden auch abgekürzt als **"QM-6")** 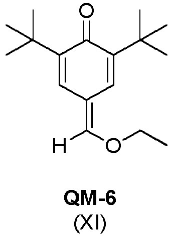 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer achten ganz besonders bevorzugten Ausführungsform des erfindungsgemäßen Verfahrens ist in der Verbindung der Struktur (I) R¹ und R² = *tert-*Butyl und
R³ = -OCH₂CH₂CH₃, wobei die Verbindung der Struktur (I) dann die Struktur (XII) aufweist (im Folgenden auch abgekürzt als **"QM-7")** 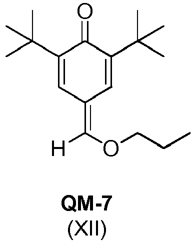 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer neunten ganz besonders bevorzugten Ausführungsform des erfindungsgemäßen Verfahrens ist in der Verbindung der Struktur (I) R¹ und R² = *tert-*Butyl und R³ = -OCH(CH₃)₂, wobei die Verbindung der Struktur (I) dann die Struktur (XIII) aufweist (im Folgenden auch abgekürzt als **"QM-8")** 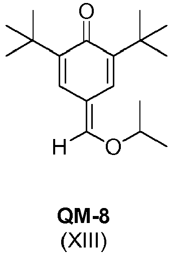 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zehnten ganz besonders bevorzugten Ausführungsform des erfindungsgemäßen Verfahrens ist in der Verbindung der Struktur (I) R¹ und R² = *tert-*Butyl und
R³ = -OCH₂CH₂CH₂CH₃, wobei die Verbindung der Struktur (I) dann die Struktur (XIV) aufweist (im Folgenden auch abgekürzt als **"QM-9")** 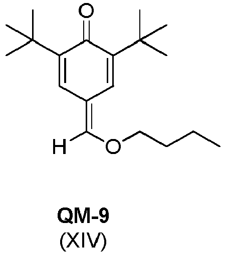 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer elften ganz besonders bevorzugten Ausführungsform des erfindungsgemäßen Verfahrens ist in der Verbindung der Struktur (I) R¹ und R² = *tert-*Butyl und
R³ = -OCH₂CH₂CH₂CH₂CH₃, wobei die Verbindung der Struktur (I) dann die Struktur (XV) aufweist (im Folgenden auch abgekürzt als **"QM-10")** 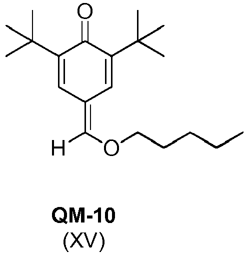 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zwölften ganz besonders bevorzugten Ausführungsform des erfindungsgemäßen Verfahrens ist in der Verbindung der Struktur (I) R¹ und R² = *tert-*Butyl und
R³ = -OCH₂CH₂CH₂CH₂CH₂CH₃, wobei die Verbindung der Struktur (I) dann die Struktur (XVI) aufweist (im Folgenden abgekürzt als **"QM-11")** 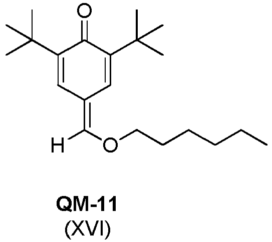 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer dreizehnten ganz besonders bevorzugten Ausführungsform des erfindungsgemäßen Verfahrens ist in der Verbindung der Struktur (I) R¹ und R² = *tert-*Butyl und
R³ = -C≡C-Phenyl, wobei die Verbindung der Struktur (I) dann die Struktur (XVII) aufweist (im Folgenden abgekürzt als **"QM-12")** 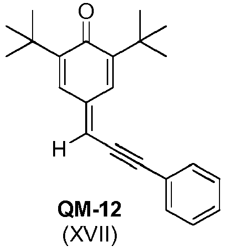 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer vierzehnten ganz besonders bevorzugten Ausführungsform des erfindungsgemäßen Verfahrens ist in der Verbindung der Struktur (I) R¹ und R² *= tert-*Butyl und R³ = -COOH, wobei die Verbindung der Struktur (I) dann die Struktur (XVIII) aufweist (im Folgenden abgekürzt als **"QM-13")** 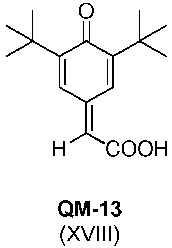 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer fünfzehnten ganz besonders bevorzugten Ausführungsform des erfindungsgemäßen Verfahrens ist in der Verbindung der Struktur (I) R¹ und R² = *tert-Butyl* und
R³ = -SCH₂CH₂CH₂CH₃, wobei die Verbindung der Struktur (I) dann die Struktur (XIX) aufweist (im Folgenden abgekürzt als **"QM-14")** 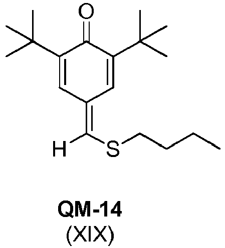 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

Im erfindungsgemäßen Verfahren kann (A) gasförmig, als Feststoff (z. B. als Pulver) oder als Flüssigkeit, insbesondere als Feststoff (z. B. als Pulver) oder Flüssigkeit, bevorzugt als Flüssigkeit eingesetzt werden. Wird (A) im erfindungsgemäßen Verfahren als Flüssigkeit eingesetzt, dann wird (A) insbesondere als Schmelze oder gelöst in (C) eingesetzt, wobei "(C)" die Bedeutung "mindestens ein Lösungsmittel" hat.

Als Lösungsmittel im erfindungsgemäßen Verfahren eignen sich alle Stoffe, in denen (A) im gewünschten Konzentrationsbereich löslich ist, und die sowohl mit (A) verträglich sind als auch keinen störenden Einfluss auf das erfindungsgemäße Verfahren haben, insbesondere unpolare, bevorzugt unpolare aromatische oder aliphatische Lösungsmittel. Bevorzugter ist das Lösungsmittel im erfindungsgemäßen Verfahren ausgewählt aus der Gruppe bestehend aus Benzol, einfach oder mehrfach alkylierten Aromaten, Alkanen mit einer Kohlenstoffzahl von 6 bis 15, Cycloalkanen mit einer Kohlenstoffzahl von 6 bis 15, hochsiedenden Kohlenwasserstoffschnitten, Ethern mit einer Kohlenstoffzahl von 6 bis 15, Estern mit einer Kohlenstoffzahl von 6 bis 15. Noch bevorzugter ist das Lösungsmittel im erfindungsgemäßen Verfahren ausgewählt aus der Gruppe bestehend aus Benzol, Toluol, Ethylbenzol, Xylol, Styrol, hochsiedende aromatische Kohlenwasserstoffschnitte. Besonders bevorzugt ist das Lösungsmittel im erfindungsgemäßen Verfahren ausgewählt aus der Gruppe bestehend aus Toluol, Ethylbenzol, Xylol und Styrol. Es kann in einer alternativen Ausführungsform auch die Cyclopentadienverbindung selbst als Lösungsmittel im erfindungsgemäßen Verfahren dienen.

Wird im erfindungsgemäßen Verfahren (A) gelöst in (C) eingesetzt, so liegt bevorzugt in der Lösung (AC) das Verhältnis (m/m) des Gesamtgewichts aller Verbindungen der Struktur (I) in der Lösung (AC) zum Gesamtgewicht aller Lösungsmittel in der Lösung (AC) im Bereich von 1 : 1000 bis 100 : 1, bevorzugter im Bereich von 1 : 100 bis 10 : 1, noch bevorzugter im Bereich von 1 : 10 bis 3 : 1.

(B) kann im erfindungsgemäßen Verfahren gasförmig, als Flüssigkeit oder als Feststoff, insbesondere gasförmig oder als Flüssigkeit, bevorzugt als Flüssigkeit vorliegen. Liegt (B) als Flüssigkeit vor, ist es noch bevorzugter, dass es als Schmelze oder in Lösung vorliegt. Besonders bevorzugt liegt (B) in einer Lösung vor. Eine solche Lösung ist in einer ersten ganz besonders bevorzugten Ausführungsform ein Prozessstrom, wie er bei Crackingprozessen erhalten wird. In einem solchen Prozessstrom liegt (B) typischerweise mit einem Anteil im Bereich von 0.0001 Gew.-% bis 15 Gew.-% vor.
In einer alternativen zweiten ganz besonders bevorzugten Ausführungsform kann die Lösung ein Prozessstrom sein, wie er bei der Herstellung des DCPD und/oder CPD selbst anfällt. In einem solchen Prozessstrom liegt (B) typischerweise mit einem Anteil zwischen 15 und 100 Gew.-% vor, bevorzugt zwischen 70 und 100 Gew-%, noch bevorzugter zwischen 70 und 99.99 Gew.-% vor.

Der Ausdruck "(A) und (B) in Kontakt bringen" bedeutet im Sinne der Erfindung insbesondere, dass (A) zu (B) gegeben wird oder (B) zu (A) gegeben wird.
Die Zugabe von (A) zu (B) oder von (B) zu (A) kann nach den gängigen Methoden des Standes der Technik erfolgen.

Vorteilhafterweise kann die Zugabe von (A) im erfindungsgemäßen Verfahren in jeglichen Zulaufstrom (feed-stream) oder Ableitung einer Destillationskolonne, in die Zu- und Ableitung eines Wärmetauschers oder in die Zu- und Ableitung eines Verdampfers ("Aufkochers") oder in die Zu- und Ableitung eines Kondensators oder in die Zu- und Ableitung eines Reaktors zugegeben werden. Darüber hinaus kann im erfindungsgemäßen Verfahren (A) auch zu Lagertanks, die einen (B) enthaltenden Prozessstrom enthalten, hinzugefügt werden. Die Zugabe von (A), kann sowohl vor als auch während eines Verfahrens, wie beispielsweise Herstellungs- oder Reinigungsverfahrens, zu (B) erfolgen.

Im erfindungsgemäßen Verfahren wird bevorzugt eine effektive Menge von (A) zugegeben. Unter dem Begriff "effektive Menge von (A)" wird im Rahmen dieser Erfindung die Menge an (A) verstanden, die notwendig ist, um die unerwünschte Polymerisation von (B) zu verzögern bzw. zu verhindern. Diese effektive Menge ist abhängig von den Bedingungen, unter denen die Cyclopentadienverbindung oder Mischung aus mehreren Cyclopentadienverbindungen gelagert oder gehandhabt wird und kann leicht vom Fachmann von Fall zu Fall bestimmt werden. Beispielsweise ist beim Cracken von Dicyclopentadien auf Grund der höheren Temperaturen eine höhere Menge an (A) notwendig als bei der Lagerung von (B) z. B. bei Raumtemperatur.

Im erfindungsgemäßen Verfahren wird (A) bevorzugt in einer solchen Menge eingesetzt, dass die Gesamtkonzentration aller Verbindungen der Struktur (I) zwischen 10 ppb (m/m) und 100000 ppm (m/m), besonders bevorzugt zwischen 1 ppm (m/m) und 50000 ppm (m/m), ganz besonders bevorzugt zwischen 10 ppm und 10000 ppm (m/m), am bevorzugtesten zwischen 100 ppm und 5000 ppm (m/m), jeweils bezogen auf das Gesamtgewicht aller Cyclopentadienverbindungen, beträgt.

Die Temperatur, bei welcher das erfindungsgemäße Verfahren durchgeführt werden kann, ist grundsätzlich nicht beschränkt, es ist vielmehr ein Merkmal der vorliegenden Erfindung, dass das erfindungsgemäße Verfahren sowohl bei niedrigen als auch bei hohen Temperaturen, insbesondere im Bereich von 0 °C bis 250 °C, bevorzugt 0 °C bis 200 °C, durchgeführt werden kann.

Im erfindungsgemäßen Verfahren kann neben (A) auch zusätzlich ein Polymerisationsinhibitor (D) eingesetzt werden. Solche Polymerisationsinhibitoren sind im Stand der Technik beschrieben, dabei handelt es sich beispielsweise Nitroxide wie z. B. Oxo-TEMPO oder 4-Hydroxy-TEMPO, Phenylendiamine, Hydroxylamine wie Diethylhydroxylamin (DEHA), Nitro- oder Nitrosoaromaten wie DNBP, (Di-)Phenole wie Hydrochinon, TBC oder 2,6-Di-*tert*-Butylphenol, Benzochinone, Phenothiazine wie PTZ.

### Erfindungsgemäße Zusammensetzung

Die Erfindung betrifft auch eine Zusammensetzung (AB), umfassend (A) und (B),
wobei
(A) mindestens eine Verbindung der Struktur (I) 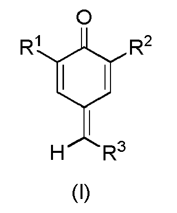 mit
   R¹ und R² sind unabhängig voneinander Wasserstoff, Alkylgruppe mit 1 bis 18 Kohlenstoffatomen, Cycloalkylgruppe mit 3 bis 15 Kohlenstoffatomen, Arylgruppe mit 6 bis 15 Kohlenstoffatomen oder Phenylalkylgruppe mit 7 bis 15 Kohlenstoffatomen;
   R³ = -CN, -COOH, -COOR⁴, -COR⁵, -OCOR⁶, -CONR⁷R⁸, -PO(OR⁹)₂, -O-R¹⁰ ,-S-R¹¹, -R¹², -C≡C-R¹³ oder Halogen;
      mit
      R⁴, R⁵, R⁶ = Alkylgruppe mit 1 bis 18 Kohlenstoffatomen, Cycloalkylgruppe mit 3 bis 12 Kohlenstoffatomen, Arylgruppe mit 6 bis 10 Kohlenstoffatomen;
      R⁷ und R⁸ sind unabhängig voneinander
         Wasserstoff;
         Alkylgruppe mit 1 bis 15 Kohlenstoffatomen, die substituiert sein kann mit mindestens einem Substituenten ausgewählt aus der Gruppe bestehend aus Alkylaminogruppe mit 1 bis 4 Kohlenstoffatomen, Dialkylaminogruppe mit 2 bis 8 Kohlenstoffatomen, Hydroxygruppe;
         Phenylalkylgruppe mit 7 bis 15 Kohlenstoffatomen, die substituiert sein kann mit mindestens einem Substituenten ausgewählt aus der Gruppe bestehend aus Hydroxygruppe, Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen;
         Arylgruppe mit 6 bis 10 Kohlenstoffatomen, die substituiert sein kann mit mindestens einem Substituenten ausgewählt aus der Gruppe bestehend aus Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen, Hydroxygruppe;
         oder NR⁷R⁸ ist Morpholino, Piperidino oder Pyrrolidino;
      R⁹, R¹⁰, R¹¹ =
         Wasserstoff;
         Alkylgruppe mit 1 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[o(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Cycloalkylgruppe mit 3 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴,
            -[o(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Phenylalkylgruppe mit 7 bis 15 Kohlenstoffatomen die substituiert sein kann mit mindestens einem Substituenten ausgewählt aus der Gruppe bestehend aus Hydroxygruppe, Dialkylaminogruppe, -OR¹⁴,
            -[o(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen; oder
         Arylgruppe mit 6 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴,
            -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
      R¹² = 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen; wobei die Reste 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
      R¹³ = Wasserstoff, Alkylgruppe mit 1 bis 12 Kohlenstoffatomen, Arylgruppe mit 6 bis 10 Kohlenstoffatomen, wobei die Arylgruppe mit 6 bis 10 Kohlenstoffatomen substituiert sein kann mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
      wobei die Substituenten vom Typ R¹, R², R³ gleich oder unterschiedlich sind,
      ist,
      und
(B) mindestens eine Cyclopentadienverbindung
   ist.

In einer bevorzugten Ausführungsform der erfindungsgemäßen Zusammensetzung (AB) sind R¹ und R² in der Verbindung der Struktur (I) unabhängig voneinander ausgewählt aus der Gruppe bestehend aus Methyl, Ethyl, *n*-Propyl, *iso*-Propyl, *n*-Butyl, *iso*-Butyl und *tert*-Butyl; und
R³ = -CN, -COOH, -COOR⁴, -COR⁵, -OCOR⁶, -CONR⁷R⁸, -PO(OR⁹)₂, -O-R¹⁰ ,-S-R¹¹, -R¹² -C≡C-R¹³ oder Halogen;
mit
R⁴, R⁵, R⁶ = Alkylgruppe mit 1 bis 8 Kohlenstoffatomen oder Phenyl;
R⁷R⁸ sind unabhängig voneinander Wasserstoff oder eine Alkylgruppe mit 1 bis 4 Kohlenstoffatomen, oder NR⁷R⁸ ist Morpholino oder Piperidino;
R⁹, R¹⁰, R¹¹ = Alkylgruppe mit 1 bis 8 Kohlenstoffatomen oder Phenyl;
R¹² = 2-Furylgruppe, 3-Furylgruppe oder Arylgruppe mit 6 bis 15 Kohlenstoffatomen, wobei die Reste 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Alkylgruppe mit 1 bis 8 Kohlenstoffatomen;
R¹³ = Wasserstoff, Alkylgruppe mit 1 bis 12 Kohlenstoffatomen, Arylgruppe mit 6 bis 10 Kohlenstoffatomen.

In einer bevorzugteren Ausführungsform der erfindungsgemäßen Zusammensetzung (AB) sind R¹ und R² in der Verbindung der Struktur (I) unabhängig voneinander ausgewählt aus der Gruppe bestehend aus Methyl und *tert*-Butyl; und
R³ = -CN, -COOH, -COOR⁴, -O-R¹⁰, -S-R¹¹, -R¹², -C≡C-R¹³ oder Halogen;
mit
R⁴ = Alkylgruppe mit 1 bis 4 Kohlenstoffatomen;
R¹⁰, R¹¹ = Alkylgruppe mit 1 bis 6 Kohlenstoffatomen;
R¹² = 2-Furylgruppe, 3-Furylgruppe oder Arylgruppe mit 6 bis 12 Kohlenstoffatomen, wobei die Reste 2-Furylgruppe, 3-Furylgruppe oder Arylgruppe mit 6 bis 12 Kohlenstoffatomen substituiert sein können mit mindestens einem Substituenten ausgewählt aus der Gruppe bestehend aus Hydroxygruppe, Alkylgruppe mit 1 bis 8 Kohlenstoffatomen;
R¹³ = Arylgruppe mit 6 bis 10 Kohlenstoffatomen.

In einer noch bevorzugteren Ausführungsform der erfindungsgemäßen Zusammensetzung (AB) sind R¹ und R² in der Verbindung der Struktur (I) *tert*-Butyl; und
R³ = -CN, -COOH, -O-R¹⁰, -S-R¹¹, -R¹² oder -C≡C-R¹³,
mit
R¹⁰ = Methyl, Ethyl, *iso*-Propyl, *n*-Propyl, *n*-Butyl, *iso*-Butyl, sec-Butyl, tert-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl, bevorzugt Methyl, Ethyl, *iso*-Propyl, *n*-Propyl, *sec*-Butyl, *n*-Butyl, *n*-Pentyl oder *n*-Hexyl;
R¹¹ = Alkylgruppe mit 1 bis 6 Kohlenstoffatomen;
R¹² = 2-Furylgruppe, 3-Furylgruppe oder Phenyl, wobei Phenyl substituiert sein kann mit mindestens einem Substituenten ausgewählt aus der Gruppe bestehend aus Hydroxygruppe, Alkylgruppe mit 1 bis 8 Kohlenstoffatomen;
R¹³ = Phenyl.

In einer ersten ganz besonders bevorzugten Ausführungsform der erfindungsgemäßen Zusammensetzung (AB) ist in der Verbindung der Struktur (I) R¹ und R² = *tert*-Butyl und R³ = CN, wobei die Verbindung der Struktur (I) dann die Struktur (V) aufweist (im Folgenden auch abgekürzt als **"QM-1"**) 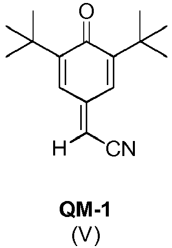 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zweiten ganz besonders bevorzugten Ausführungsform der erfindungsgemäßen Zusammensetzung (AB) ist in der Verbindung der Struktur (I) R¹ und R² = *tert-*Butyl und R³= Phenyl, wobei die Verbindung der Struktur (I) dann die Struktur (VI) aufweist (im Folgenden auch abgekürzt als **"QM-2")** 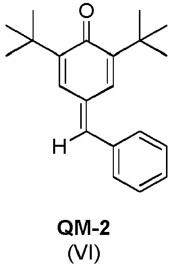 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer dritten ganz besonders bevorzugten Ausführungsform der erfindungsgemäßen Zusammensetzung (AB) ist in der Verbindung der Struktur (I) R¹ und R² = *tert-*Butyl und R³ = 3,5-Di-*tert*-Butyl-4-Hydroxy-Phenyl, wobei die Verbindung der Struktur (I) dann die Struktur (VII) aufweist (im Folgenden auch abgekürzt als **"QM-3")** 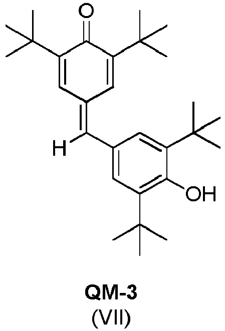 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer vierten ganz besonders bevorzugten Ausführungsform der erfindungsgemäßen Zusammensetzung (AB) ist in der Verbindung der Struktur (I) R¹ und R² = *tert-*Butyl und R³ = 2-Furyl, wobei die Verbindung der Struktur (I) dann die Struktur (VIII) aufweist (im Folgenden auch abgekürzt als **"QM-4")** 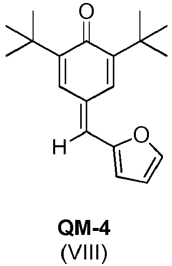 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer fünften ganz besonders bevorzugten Ausführungsform der erfindungsgemäßen Zusammensetzung (AB) ist in der Verbindung der Struktur (I) R¹ und R² = *tert*-Butyl und R³ = -OR¹⁰, wobei

R¹⁰ = Methyl, Ethyl, *iso*-Propyl, *n*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl, bevorzugt Methyl, Ethyl, *iso*-Propyl, *n*-Propyl, *n*-Butyl, *sec*-Butyl, *tert*-Butyl, *n*-Pentyl oder *n*-Hexyl, besonders bevorzugt Methyl, Ethyl, *n*-Propyl, *iso*-Propyl, *n*-Butyl, *n*-Pentyl oder *n*-Hexyl;
wobei die Verbindung der Struktur (I) dann die Struktur (IX) aufweist 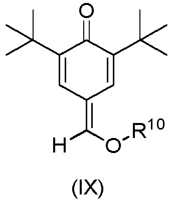 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer sechsten ganz besonders bevorzugten Ausführungsform der erfindungsgemäßen Zusammensetzung (AB) ist in der Verbindung der Struktur (I) R¹ und R² = *tert-*Butyl und R³ = -OCH₃, wobei die Verbindung der Struktur (I) dann die Struktur (X) aufweist (im Folgenden auch abgekürzt als **"QM-5"**) 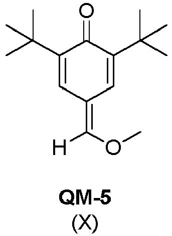 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer siebten ganz besonders bevorzugten Ausführungsform der erfindungsgemäßen Zusammensetzung (AB) ist in der Verbindung der Struktur (I) R¹ und R² = *tert-*Butyl und R³ = -OCH₂CH₃, wobei die Verbindung der Struktur (I) dann die Struktur (XI) aufweist (im Folgenden auch abgekürzt als **"QM-6")** 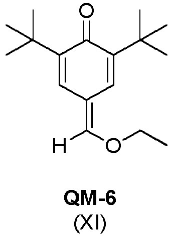 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer achten ganz besonders bevorzugten Ausführungsform der erfindungsgemäßen Zusammensetzung (AB) ist in der Verbindung der Struktur (I) R¹ und R² = *tert-*Butyl und R³ = -OCH₂CH₂CH₃, wobei die Verbindung der Struktur (I) dann die Struktur (XII) aufweist (im Folgenden auch abgekürzt als **"QM-7")** 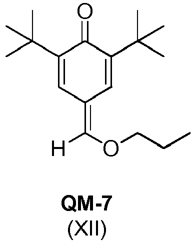 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer neunten ganz besonders bevorzugten Ausführungsform der erfindungsgemäßen Zusammensetzung (AB) ist in der Verbindung der Struktur (I) R¹ und R² = *tert-*Butyl und R³ = -OCH(CH₃)₂, wobei die Verbindung der Struktur (I) dann die Struktur (XIII) aufweist (im Folgenden auch abgekürzt als **"QM-8")** 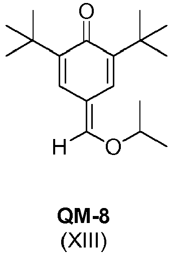 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zehnten ganz besonders bevorzugten Ausführungsform der erfindungsgemäßen Zusammensetzung (AB) ist in der Verbindung der Struktur (I) R¹ und R² = *tert-*Butyl und R³ = -OCH₂CH₂CH₂CH₃, wobei die Verbindung der Struktur (I) dann die Struktur (XIV) aufweist (im Folgenden auch abgekürzt als **"QM-9")** 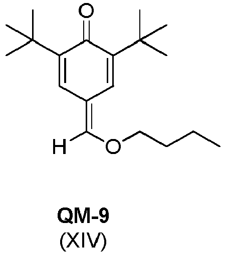 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer elften ganz besonders bevorzugten Ausführungsform der erfindungsgemäßen Zusammensetzung (AB) ist in der Verbindung der Struktur (I) R¹ und R² = *tert-*Butyl und R³ = -OCH₂CH₂CH₂CH₂CH₃, wobei die Verbindung der Struktur (I) dann die Struktur (XV) aufweist (im Folgenden auch abgekürzt als **"QM-10")** 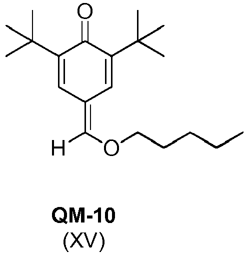 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zwölften ganz besonders bevorzugten Ausführungsform der erfindungsgemäßen Zusammensetzung (AB) ist in der Verbindung der Struktur (I) R¹ und R² = *tert-*Butyl und R³ = -OCH₂CH₂CH₂CH₂CH₂CH₃, wobei die Verbindung der Struktur (I) dann die Struktur (XVI) aufweist (im Folgenden abgekürzt als **"QM-11")** 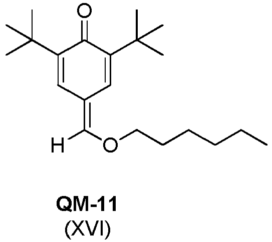 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer dreizehnten ganz besonders bevorzugten Ausführungsform der erfindungsgemäßen Zusammensetzung (AB) ist in der Verbindung der Struktur (I) R¹ und R² = *tert-* und R³ = -C≡C-Phenyl, wobei die Verbindung der Struktur (I) dann die Struktur (XVII) aufweist (im Folgenden abgekürzt als **"QM-12")** 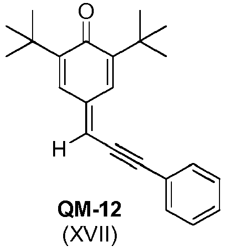 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer vierzehnten ganz besonders bevorzugten Ausführungsform der erfindungsgemäßen Zusammensetzung (AB) ist in der Verbindung der Struktur (I) R¹ und R² = *tert-*Butyl und R³ = -COOH, wobei die Verbindung der Struktur (I) dann die Struktur (XVIII) aufweist (im Folgenden abgekürzt als **"QM-13")** 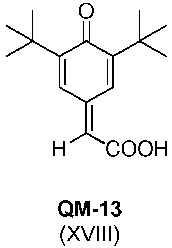 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer fünfzehnten ganz besonders bevorzugten Ausführungsform der erfindungsgemäßen Zusammensetzung (AB) ist in der Verbindung der Struktur (I) R¹ und R² = *tert-*Butyl und R³ = -SCH₂CH₂CH₂CH₃, wobei die Verbindung der Struktur (I) dann die Struktur (XIX) aufweist (im Folgenden abgekürzt als **"QM-14")** 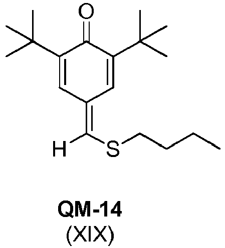 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In der erfindungsgemäßen Zusammensetzung (AB) liegt bevorzugt die Gesamtkonzentration aller Verbindungen der Struktur (I) in der Zusammensetzung (AB) zwischen 10 ppb (m/m) und 100000 ppm (m/m), besonders bevorzugt zwischen 1 ppm (m/m) und 50000 ppm (m/m), ganz besonders bevorzugt zwischen 10 ppm und 10000 ppm (m/m), am bevorzugtesten zwischen 100 ppm und 5000 ppm (m/m), jeweils bezogen auf das Gesamtgewicht aller Cyclopentadienverbindungen in der Zusammensetzung (AB).

Die erfindungsgemäßen Zusammensetzung (AB) kann in einer weiteren bevorzugten Ausführungsform auch zusätzlich (C) umfassen, wobei "(C)" "mindestens ein Lösungsmittel" bedeutet.

Als Lösungsmittel der erfindungsgemäßen Zusammensetzung (AB) eignen sich alle Stoffe, in denen (A) im gewünschten Konzentrationsbereich löslich ist, und die sowohl mit (A) verträglich sind als auch keinen störenden Einfluss auf das erfindungsgemäße Verfahren haben, insbesondere unpolare, bevorzugt unpolare aromatische oder aliphatische Lösungsmittel. Bevorzugter ist das Lösungsmittel der erfindungsgemäßen Zusammensetzung (AB) ausgewählt aus der Gruppe bestehend aus Benzol, einfach oder mehrfach alkylierten Aromaten, Alkanen mit einer Kohlenstoffzahl von 6 bis 15, Cycloalkanen mit einer Kohlenstoffzahl von 6 bis 15, hochsiedenden Kohlenwasserstoffschnitten, Ethern mit einer Kohlenstoffzahl von 6 bis 15, Estern mit einer Kohlenstoffzahl von 6 bis 15. Noch bevorzugter ist das Lösungsmittel der erfindungsgemäßen Zusammensetzung (AB) ausgewählt aus der Gruppe bestehend aus Benzol, Toluol, Ethylbenzol, Xylol, Styrol, hochsiedende aromatische Kohlenwasserstoffschnitte. Besonders bevorzugt ist das Lösungsmittel der erfindungsgemäßen Zusammensetzung (AB) ausgewählt aus der Gruppe bestehend aus Toluol, Ethylbenzol, Xylol und Styrol. Es kann in einer alternativen Ausführungsform auch die Cyclopentadienverbindung selbst als Lösungsmittel der erfindungsgemäßen Zusammensetzung (AB) dienen.

Umfasst die erfindungsgemäße Zusammensetzung (AB) auch (C), so liegt dann bevorzugt in der Zusammensetzung (AB) das Verhältnis (m/m) des Gesamtgewichts aller von der Zusammensetzung (AB) umfasster Verbindungen der Struktur (I) zum Gesamtgewicht aller von der Zusammensetzung (AB) umfasster Lösungsmittel im Bereich von 1 : 1000 bis 100 : 1, bevorzugter im Bereich von 1 : 100 bis 10 : 1, noch bevorzugter im Bereich von 1 : 10 bis 3 : 1 liegt.

### Erfindungsgemäße Verwendung

Der Ausdruck "erfindungsgemäße Verwendung" ist gleichbedeutend mit "Verwendung von (A) zur Inhibierung der Polymerisation von (B)".

Die Erfindung betrifft auch die Verwendung von (A) zur Inhibierung der Polymerisation von (B),
wobei
(A) mindestens eine Verbindung der Struktur (I) 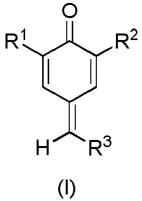 mit
   R¹ und R² sind unabhängig voneinander Wasserstoff, Alkylgruppe mit 1 bis 18 Kohlenstoffatomen, Cycloalkylgruppe mit 3 bis 15 Kohlenstoffatomen, Arylgruppe mit 6 bis 15 Kohlenstoffatomen oder Phenylalkylgruppe mit 7 bis 15 Kohlenstoffatomen; R³ = -CN, -COOH, -COOR⁴, -COR⁵, -OCOR⁶, -CONR⁷R⁸, -PO(OR⁹)₂, -O-R¹⁰ ,-S-R¹¹, -R¹², -C≡C-R¹³ oder Halogen;
      mit
      R⁴, R⁵, R⁶ = Alkylgruppe mit 1 bis 18 Kohlenstoffatomen, Cycloalkylgruppe mit 3 bis 12 Kohlenstoffatomen, Arylgruppe mit 6 bis 10 Kohlenstoffatomen;
      R⁷ und R⁸ sind unabhängig voneinander
         Wasserstoff;
         Alkylgruppe mit 1 bis 15 Kohlenstoffatomen, die substituiert sein kann mit mindestens einem Substituenten ausgewählt aus der Gruppe bestehend aus Alkylaminogruppe mit 1 bis 4 Kohlenstoffatomen, Dialkylaminogruppe mit 2 bis 8 Kohlenstoffatomen, Hydroxygruppe;
         Phenylalkylgruppe mit 7 bis 15 Kohlenstoffatomen, die substituiert sein kann mit mindestens einem Substituenten ausgewählt aus der Gruppe bestehend aus Hydroxygruppe, Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen;
         Arylgruppe mit 6 bis 10 Kohlenstoffatomen, die substituiert sein kann mit mindestens einem Substituenten ausgewählt aus der Gruppe bestehend aus Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen, Hydroxygruppe;
         oder NR⁷R⁸ ist Morpholino, Piperidino oder Pyrrolidino;
      R⁹, R¹⁰, R¹¹ =
         Wasserstoff;
         Alkylgruppe mit 1 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴,
         -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Cycloalkylgruppe mit 3 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
         Phenylalkylgruppe mit 7 bis 15 Kohlenstoffatomen die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen; oder
         Arylgruppe mit 6 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
      R¹² = 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen; wobei die Reste 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
      R¹³ = Wasserstoff, Alkylgruppe mit 1 bis 12 Kohlenstoffatomen, Arylgruppe mit 6 bis 10 Kohlenstoffatomen, wobei die Arylgruppe mit 6 bis 10 Kohlenstoffatomen substituiert sein kann mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
   wobei die Substituenten vom Typ R¹, R², R³ gleich oder unterschiedlich sind,
   ist,
   und
(B) mindestens eine Cyclopentadienverbindung
   ist.

In einer bevorzugten Ausführungsform der erfindungsgemäßen Verwendung sind R¹ und R² in der Verbindung der Struktur (I) unabhängig voneinander ausgewählt aus der Gruppe bestehend aus Methyl, Ethyl, *n*-Propyl, *iso*-Propyl, *n*-Butyl, *iso*-Butyl und *tert*-Butyl; und R³ = -CN, -COOH, -COOR⁴, -COR⁵, -OCOR⁶, -CONR⁷R⁸, -PO(OR⁹)₂, -O-R¹⁰ ,-S-R¹¹, -R¹² -C≡C-R¹³ oder Halogen;
mit
R⁴, R⁵, R⁶ = Alkylgruppe mit 1 bis 8 Kohlenstoffatomen oder Phenyl;
R⁷R⁸ sind unabhängig voneinander Wasserstoff oder eine Alkylgruppe mit 1 bis 4 Kohlenstoffatomen, oder NR⁷R⁸ ist Morpholino oder Piperidino;
R⁹, R¹⁰, R¹¹ = Alkylgruppe mit 1 bis 8 Kohlenstoffatomen oder Phenyl;
R¹² = 2-Furylgruppe, 3-Furylgruppe oder Arylgruppe mit 6 bis 15 Kohlenstoffatomen, wobei die Reste 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Alkylgruppe mit 1 bis 8 Kohlenstoffatomen;
R¹³ = Wasserstoff, Alkylgruppe mit 1 bis 12 Kohlenstoffatomen, Arylgruppe mit 6 bis 10 Kohlenstoffatomen.

In einer bevorzugteren Ausführungsform der erfindungsgemäßen Verwendung sind R¹ und R² in der Verbindung der Struktur (I) unabhängig voneinander ausgewählt aus der Gruppe bestehend aus Methyl und *tert*-Butyl; und
R³ = -CN, -COOH, -COOR⁴, -O-R¹⁰, -S-R¹¹, -R¹², -C≡C-R¹³ oder Halogen;
mit
R⁴ = Alkylgruppe mit 1 bis 4 Kohlenstoffatomen;
R¹⁰, R¹¹ = Alkylgruppe mit 1 bis 6 Kohlenstoffatomen;
R¹² = 2-Furylgruppe, 3-Furylgruppe oder Arylgruppe mit 6 bis 12 Kohlenstoffatomen, wobei die Reste 2-Furylgruppe, 3-Furylgruppe oder Arylgruppe mit 6 bis 12 Kohlenstoffatomen substituiert sein können mit mindestens einem Substituenten ausgewählt aus der Gruppe bestehend aus Hydroxygruppe, Alkylgruppe mit 1 bis 8 Kohlenstoffatomen;
R¹³ = Arylgruppe mit 6 bis 10 Kohlenstoffatomen.

In einer noch bevorzugteren Ausführungsform der erfindungsgemäßen Verwendung sind R¹ und R² in der Verbindung der Struktur (I) *tert*-Butyl; und
R³ = -CN, -COOH, -O-R¹⁰, -S-R¹¹, -R¹² oder -C≡C-R¹³,
mit
R¹⁰ = Methyl, Ethyl, *iso*-Propyl, *n*-Propyl, *n*-Butyl, *iso*-Butyl, sec-Butyl, tert-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl, bevorzugt Methyl, Ethyl, *iso*-Propyl, *n*-Propyl, *sec*-Butyl, *n*-Butyl, *n*-Pentyl oder *n*-Hexyl;
R¹¹ = Alkylgruppe mit 1 bis 6 Kohlenstoffatomen;
R¹² = 2-Furylgruppe, 3-Furylgruppe oder Phenyl, wobei Phenyl substituiert sein kann mit mindestens einem Substituenten ausgewählt aus der Gruppe bestehend aus Hydroxygruppe, Alkylgruppe mit 1 bis 8 Kohlenstoffatomen;
R¹³ = Phenyl.

In einer ersten ganz besonders bevorzugten Ausführungsform der erfindungsgemäßen Verwendung ist in der Verbindung der Struktur (I) R¹ und R² = *tert-*Butyl und R³ = CN, wobei die Verbindung der Struktur (I) dann die Struktur (V) aufweist (im Folgenden auch abgekürzt als **"QM-1**") 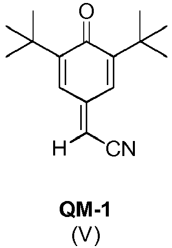 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zweiten ganz besonders bevorzugten Ausführungsform der erfindungsgemäßen Verwendung ist in der Verbindung der Struktur (I) R¹ und R² = *tert-*Butyl und R³ = Phenyl, wobei die Verbindung der Struktur (I) dann die Struktur (VI) aufweist (im Folgenden auch abgekürzt als **"QM-2"**) 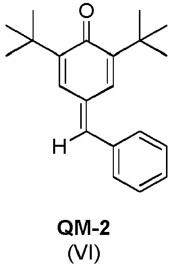 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer dritten ganz besonders bevorzugten Ausführungsform der erfindungsgemäßen Verwendung ist in der Verbindung der Struktur (I) R¹ und R² = *tert-*Butyl und R³ = 3,5-Di-*tert-*Butyl-4-Hydroxy-Phenyl, wobei die Verbindung der Struktur (I) dann die Struktur (VII) aufweist (im Folgenden auch abgekürzt als **"QM-3")** 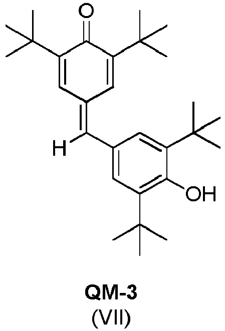 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer vierten ganz besonders bevorzugten Ausführungsform der erfindungsgemäßen Verwendung ist in der Verbindung der Struktur (I) R¹ und R² = *tert-*Butyl und R³ = 2-Furyl, wobei die Verbindung der Struktur (I) dann die Struktur (VIII) aufweist (im Folgenden auch abgekürzt als **"QM-4")** 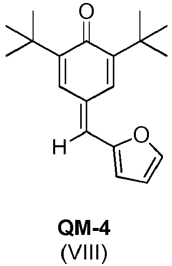 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer fünften ganz besonders bevorzugten Ausführungsform der erfindungsgemäßen Verwendung ist in der Verbindung der Struktur (I) R¹ und R² = *tert*-Butyl und R³ = -OR¹⁰, wobei
R¹⁰ = Methyl, Ethyl, *iso*-Propyl, *n*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl, bevorzugt Methyl, Ethyl, *iso*-Propyl, *n*-Propyl, *n*-Butyl, *sec*-Butyl, *tert*-Butyl, *n*-Pentyl oder *n*-Hexyl, besonders bevorzugt Methyl, Ethyl, *n*-Propyl, *iso*-Propyl, *n*-Butyl, *n*-Pentyl oder *n*-Hexyl;
wobei die Verbindung der Struktur (I) dann die Struktur (IX) aufweist 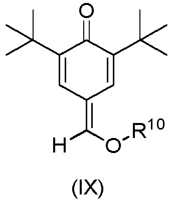 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer sechsten ganz besonders bevorzugten Ausführungsform der erfindungsgemäßen Verwendung ist in der Verbindung der Struktur (I) R¹ und R² = *tert-*Butyl und R³ = -OCH₃, wobei die Verbindung der Struktur (I) dann die Struktur (X) aufweist (im Folgenden auch abgekürzt als **"QM-5")** 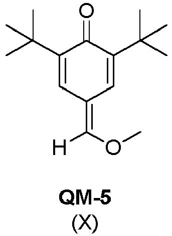 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer siebten ganz besonders bevorzugten Ausführungsform der erfindungsgemäßen Verwendung ist in der Verbindung der Struktur (I) R¹ und R² = *tert*-Butyl und R³ = -OCH₂CH₃, wobei die Verbindung der Struktur (I) dann die Struktur (XI) aufweist (im Folgenden auch abgekürzt als **"QM-6")** 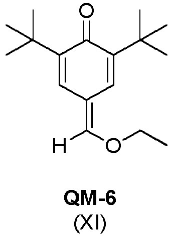 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer achten ganz besonders bevorzugten Ausführungsform der erfindungsgemäßen Verwendung ist in der Verbindung der Struktur (I) R¹ und R² *= tert-*Butul und R³ = -OCH₂CH₂CH₃, wobei die Verbindung der Struktur (I) dann die Struktur (XII) aufweist (im Folgenden auch abgekürzt als **"QM-7")** 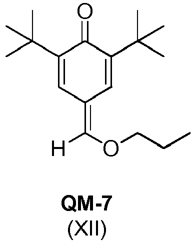 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer neunten ganz besonders bevorzugten Ausführungsform der erfindungsgemäßen Verwendung ist in der Verbindung der Struktur (I) R¹ und R² = *tert-*Butyl und R³ = -OCH(CH₃)₂, wobei die Verbindung der Struktur (I) dann die Struktur (XIII) aufweist (im Folgenden auch abgekürzt als **"QM-8")** 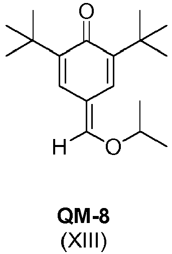 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zehnten ganz besonders bevorzugten Ausführungsform der erfindungsgemäßen Verwendung ist in der Verbindung der Struktur (I) R¹ und R² = *tert-*Butyl und R³ = -OCH₂CH₂CH₂CH₃, wobei die Verbindung der Struktur (I) dann die Struktur (XIV) aufweist (im Folgenden auch abgekürzt als **"QM-9")** 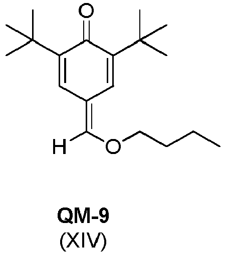 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer elften ganz besonders bevorzugten Ausführungsform der erfindungsgemäßen Verwendung ist in der Verbindung der Struktur (I) R¹ und R² = *tert-*Butyl und R³ = -OCH₂CH₂CH₂CH₂CH₃, wobei die Verbindung der Struktur (I) dann die Struktur (XV) aufweist (im Folgenden auch abgekürzt als **"QM-10")** 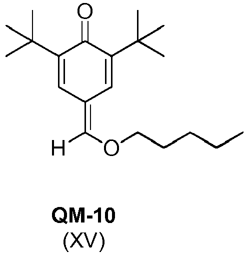 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer zwölften ganz besonders bevorzugten Ausführungsform der erfindungsgemäßen Verwendung ist in der Verbindung der Struktur (I) R¹ und R² = *tert-*Butyl und R³ = -OCH₂CH₂CH₂CH₂CH₂CH₃, wobei die Verbindung der Struktur (I) dann die Struktur (XVI) aufweist (im Folgenden abgekürzt als "**QM-11**") 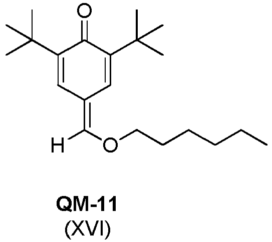 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer dreizehnten ganz besonders bevorzugten Ausführungsform der erfindungsgemäßen Verwendung ist in der Verbindung der Struktur (I) R¹ und R² = *tert-*Butyl und R³ = -C≡C-Phenyl, wobei die Verbindung der Struktur (I) dann die Struktur (XVII) aufweist (im Folgenden abgekürzt als "**QM-12**") 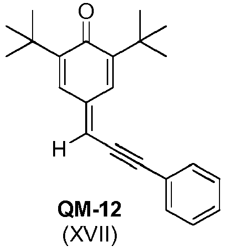 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer vierzehnten ganz besonders bevorzugten Ausführungsform der erfindungsgemäßen Verwendung ist in der Verbindung der Struktur (I) R¹ und R² = *tert-*Butyl und R³ = -COOH, wobei die Verbindung der Struktur (I) dann die Struktur (XVIII) aufweist (im Folgenden abgekürzt als "**QM-13**") 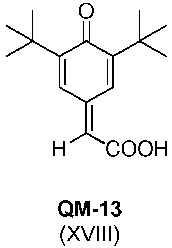 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

In einer fünfzehnten ganz besonders bevorzugten Ausführungsform der erfindungsgemäßen Verwendung ist in der Verbindung der Struktur (I) R¹ und R² = *tert-*Butyl und R³ = -SCH₂CH₂CH₂CH₃, wobei die Verbindung der Struktur (I) dann die Struktur (XIX) aufweist (im Folgenden abgekürzt als "**QM-14**") 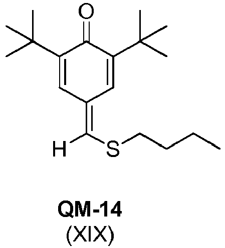 und die Cyclopentadienverbindung ist aus der Gruppe bestehend aus Cyclopentadien, Dicyclopentadien, alkyliertem Cyclopentadien, alkyliertem Dicyclopentadien ausgewählt, besonders bevorzugt aus der Gruppe bestehend aus Cyclopentadien und Dicyclopentadien ausgewählt.

Bei der erfindungsgemäßen Verwendung kann (A) gasförmig, als Feststoff (z. B. als Pulver) oder als Flüssigkeit, insbesondere als Feststoff (z.B. als Pulver) oder Flüssigkeit, bevorzugt als Flüssigkeit eingesetzt werden. Wird (A) bei der erfindungsgemäßen Verwendung als Flüssigkeit eingesetzt, dann wird (A) insbesondere als Schmelze oder gelöst in (C) eingesetzt, wobei "(C)" die Bedeutung "mindestens ein Lösungsmittel" hat.

Als Lösungsmittel bei der erfindungsgemäßen Verwendung eignen sich alle Stoffe, in denen (A) im gewünschten Konzentrationsbereich löslich ist, und die sowohl mit (A) verträglich sind als auch keinen störenden Einfluss auf die erfindungsgemäße Verwendung haben, insbesondere unpolare, bevorzugt unpolare aromatische oder aliphatische Lösungsmittel. Bevorzugter ist das Lösungsmittel bei der erfindungsgemäßen Verwendung ausgewählt aus der Gruppe bestehend aus Benzol, einfach oder mehrfach alkylierten Aromaten, Alkanen mit einer Kohlenstoffzahl von 6 bis 15, Cycloalkanen mit einer Kohlenstoffzahl von 6 bis 15, Ethern mit einer Kohlenstoffzahl von 6 bis 15, hochsiedenden Kohlenwasserstoffschnitten, Estern mit einer Kohlenstoffzahl von 6 bis 15. Noch bevorzugter ist das Lösungsmittel bei der erfindungsgemäßen Verwendung ausgewählt aus der Gruppe bestehend aus Benzol, Toluol, Ethylbenzol, Xylol, Styrol, hochsiedende aromatische Kohlenwasserstoffschnitte. Besonders bevorzugt ist das Lösungsmittel bei der erfindungsgemäßen Verwendung ausgewählt aus der Gruppe bestehend aus Toluol, Ethylbenzol, Xylol und Styrol. Es kann in einer alternativen Ausführungsform auch die Cyclopentadienverbindung selbst als Lösungsmittel bei der erfindungsgemäßen Verwendung dienen.

Wird bei der erfindungsgemäßen Verwendung (A) gelöst in (C) eingesetzt, so liegt bevorzugt in der Lösung (AC) das Verhältnis (m/m) des Gesamtgewichts aller Verbindungen der Struktur (I) in der Lösung (AC) zum Gesamtgewicht aller Lösungsmittel in der Lösung (AC) im Bereich von 1 : 1000 bis 100 : 1, bevorzugter im Bereich von 1 : 100 bis 10 : 1, noch bevorzugter im Bereich von 1 : 10 bis 3 : 1.

(B) kann bei der erfindungsgemäßen Verwendung gasförmig, als Flüssigkeit oder als Feststoff, insbesondere gasförmig oder als Flüssigkeit, bevorzugt als Flüssigkeit vorliegen. Liegt (B) als Flüssigkeit vor, ist es noch bevorzugter, dass es als Schmelze oder in Lösung vorliegt. Besonders bevorzugt liegt (B) in einer Lösung vor. Eine solche Lösung ist in einer ersten ganz besonders bevorzugten Ausführungsform der erfindungsgemäßen Verwendung ein Prozessstrom, wie er bei Crackingprozessen erhalten wird. In einem solchen Prozessstrom liegt (B) typischerweise mit einem Anteil im Bereich von 0.0001 Gew.-% bis 15 Gew.-% vor.

In einer alternativen zweiten ganz besonders bevorzugten Ausführungsform der erfindungsgemäßen Verwendung kann die Lösung ein Prozessstrom sein, wie er bei der Herstellung des DCPD und/oder CPD selbst anfällt. In einem solchen Prozessstrom liegt (B) typischerweise mit einem Anteil zwischen 15 und 100 Gew.-% vor, bevorzugt zwischen 70 und 100 Gew-%, noch bevorzugter zwischen 70 und 99.99 Gew.-% vor.

Bei der erfindungsgemäßen Verwendung werden typischerweise (A) und (B) in Kontakt gebracht, was im Sinne der Erfindung insbesondere bedeutet, dass (A) zu (B) gegeben wird oder (B) zu (A) gegeben wird.

Die Zugabe von (A) zu (B) oder von (B) zu (A) kann nach den gängigen Methoden des Standes der Technik erfolgen.

Vorteilhafterweise kann die Zugabe von (A) bei der erfindungsgemäßen Verwendung in jeglichen Zulaufstrom (feed-stream) oder Ableitung einer Destillationskolonne, in die Zu- und Ableitung eines Wärmetauschers oder in die Zu- und Ableitung eines Verdampfers ("Aufkochers") oder in die Zu- und Ableitung eines Kondensators oder in die Zu- und Ableitung eines Reaktors zugegeben werden. Darüber hinaus kann bei der erfindungsgemäßen Verwendung (A) auch zu Lagertanks, die einen (B) enthaltenden Prozessstrom enthalten, hinzugefügt werden. Die Zugabe von (A), kann sowohl vor als auch während eines Verfahrens, wie beispielsweise Herstellungs- oder Reinigungsverfahrens, zu (B) erfolgen.

Bei der erfindungsgemäßen Verwendung wird bevorzugt eine effektive Menge von (A) zugegeben.
Unter dem Begriff "effektive Menge von (A)" wird im Rahmen dieser Erfindung die Menge an (A) verstanden, die notwendig ist, um die unerwünschte Polymerisation von (B) zu verzögern bzw. zu verhindern. Diese effektive Menge ist abhängig von den Bedingungen, unter denen die Cyclopentadienverbindung oder Mischung aus mehreren Cyclopentadienverbindungen gelagert oder gehandhabt wird und kann leicht vom Fachmann von Fall zu Fall bestimmt werden. Beispielsweise ist beim Cracken von Dicyclopentadien auf Grund der höheren Temperaturen eine höhere Menge an (A) notwendig als bei der Lagerung von (B) z. B. bei Raumtemperatur.

Bei der erfindungsgemäßen Verwendung wird (A) bevorzugt in einer solchen Menge eingesetzt, dass die Gesamtkonzentration aller Verbindungen der Struktur (I) zwischen 10 ppb (m/m) und 100000 ppm (m/m), besonders bevorzugt zwischen 1 ppm (m/m) und 50000 ppm (m/m), ganz besonders bevorzugt zwischen 10 ppm und 10000 ppm (m/m), am bevorzugtesten zwischen 100 ppm und 5000 ppm (m/m), jeweils bezogen auf das Gesamtgewicht aller Cyclopentadienverbindungen, beträgt.

Die Temperatur, bei welcher die erfindungsgemäße Verwendung durchgeführt werden kann, ist grundsätzlich nicht beschränkt, es ist vielmehr ein Merkmal der vorliegenden Erfindung, dass die erfindungsgemäße Verwendung sowohl bei niedrigen als auch bei hohen Temperaturen, insbesondere im Bereich von 0 °C bis 250 °C, bevorzugt 0 °C bis 200 °C, durchgeführt werden kann.

Bei der erfindungsgemäßen Verwendung kann neben der (A) auch zusätzlich ein Polymerisationsinhibitor (D) eingesetzt werden. Solche Polymerisationsinhibitoren sind im Stand der Technik beschrieben, dabei handelt es sich beispielsweise Nitroxide wie z.B. Oxo-TEMPO oder 4-Hydroxy-TEMPO, Phenylendiamine, Hydroxylamine wie Diethylhydroxylamin (DEHA), Nitro- oder Nitrosoaromaten wie DNBP, (Di-)Phenole wie Hydrochinon, TBC oder 2,6-Di-*tert*-Butylphenol, Benzochinone, Phenothiazine wie PTZ.

Die nachfolgenden Beispiele sollen die Erfindung näher erläutern, ohne dass die Erfindung auf diese Ausführungsformen beschränkt sein soll.

### Beispielteil

### Allgemeine Beschreibung - Screeningtest; Beispiele 1 - 11

Es wird die folgende Apparatur aufgebaut: Ein 250 mL Mehrhalskolben wird mit einem Rückflusskühler, einer Stickstoffversorgung und einer Probenahme ausgerüstet.

Es werden 100 g Dicyclopentadien (Reinheit: 98%) aufgeschmolzen und in den 250 mL Kolben eingewogen.

Das Dicyclopentadien wird mit Stickstoff überströmt und es werden 50 mg (500 ppm) des in Tabelle 1 gezeigten zu testenden Inhibitors zugesetzt.

Während das Dicyclopentadien kontinuierlich weiter mit Stickstoff überströmt wird, wird der Kolben in ein Ölbad getaucht, das auf 170°C vorgeheizt wurde. Das Eintauchen des Kolbens ist der Startpunkt der Reaktion.

Beginnend mit dem Eintauchen des Kolbens werden alle 30 Minuten 0.5 - 1 mL Proben mit Hilfe einer Glasspritze entnommen. Die Proben werden im Gewichtsverhältnis 1 : 9 in Ethylbenzol verdünnt und mittels ELS-Detektor (ELS = *evaporative light scattering*) vermessen.

Der ELS-Detektor (Polymerlabs; Typ: PL-ELS 1000) ist an eine HPLC-Anlage angeschlossen, die ohne Trennsäule betrieben wird. Als Laufmittel wird Ethylbenzol mit einer Flussrate von 1 mL/min eingesetzt. Es werden 20 µL der verdünnten Probe injiziert.

Beim ELS-Detektor werden folgende Parameter eingestellt:
- Stickstoffstrom: 1.2 l/h
- Nebulizer: 100 °C
- Evaporator: 130 °C

Die detektiere Peakfläche ist ein Maß für den Oligo-/Polymergehalt der Probe. Es werden keine absoluten Oligo-Polymergehalte bestimmt. Im Messbereich ist die Peakfläche zum Oligo-/Polymergehalt proportional, so dass die Ergebnisse für die verschiedenen Inhibitoren vergleichbar sind.

Die Ergebnisse nach 120 min und 240 min sind in der folgenden Tabelle 1 zusammengefasst - in Abbildung 1 sind sämtliche Messwerte graphisch dargestellt.
Beispiel 1 ist die Blindprobe (ohne Zugabe eines Inhibitors). Beispiele 2 bis 6 sind nicht erfindungsgemäße Vergleichsbeispiele, die mit den im Stand der Technik zur Inhibierung von Cyclopentadienpolymerisationen beschriebenen Inhibitoren 4-Hydroxy-TEMPO (4-HT; Beispiel 2), 4-Butoxy-TEMPO (4-BT; Beispiel 3), *tert*-Butyl-Catechol (TBC; Beispiel 4) bzw. Dinitro-sec-Butylphenol (DNBP; Beispiel 5), Hydrochinonmonomethylether (MeHQ; Beispiel 6) durchgeführt wurden. Beispiele 7 bis 11 sind erfindungsgemäße Beispiele, die mit den Verbindungen **QM-1** (Beispiel 7), **QM-2** (Beispiel 8), **QM-5** (Beispiel 9), **QM-7** (Beispiel 10) und **QM-11** (Beispiel 11) durchgeführt wurden.

**Tabelle 1:**

| **Beispiel** | **Name und Struktur des Inhibitors** | **Peakfläche** | |
|---|---|---|---|
| | | **nach 120 min** | **nach 240 min** |
| **1** | Kein Inhibitor (Blindprobe) | 1100 | 2300 |
| **2** | 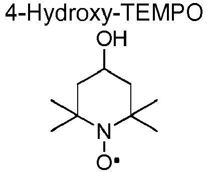 | 810 | 1880 |
| **3** | 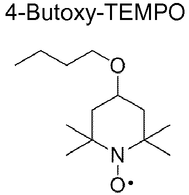 | 750 | 1830 |
| **4** | 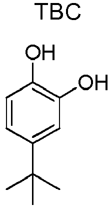 | 340 | 930 |
| **5** | 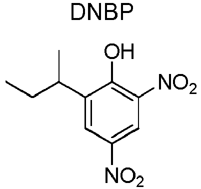 | 1140 | 2450 |
| **6** | 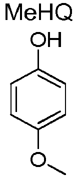 | 1110 | 2350 |
| **7** | 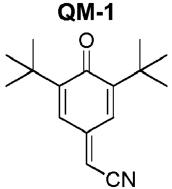 | 90 | 320 |
| **8** | 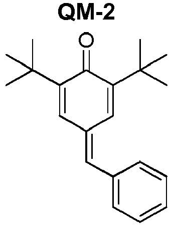 | 160 | 290 |
| **9** | 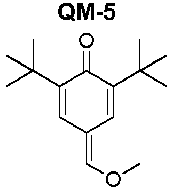 | 160 | 360 |
| **10** | 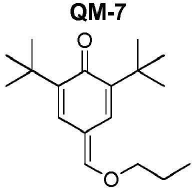 | 200 | 450 |
| **11** | 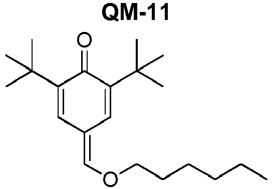 | 330 | 660 |

### Ergebnisse zu Beispielen 1 - 11:

### Beispiel 1 (nicht erfindungsgemäßes Vergleichsbeispiel): Blind-Wert (ohne Inhibitor-Zusatz)

Die Kurve steigt über den Messraum von 4 Stunden kontinuierlich an, d. h. der Polymergehalt nimmt kontinuierlich zu. Nach zwei Stunden wurde eine Peakfläche von 1100 gemessen, nach vier Stunden hatte sie sich auf 2300 etwas mehr als verdoppelt.

### Beispiele 2 & 3 (nicht erfindungsgemäße Vergleichsbeispiele): TEMPO-Derivate (4-Hydroxy-TEMPO; 4-Butoxy-TEMPO)

Wie der Kurve in Abbildung 1 zu entnehmen ist, bildet sich in den ersten 30 Minuten fast kein Polymer, danach steigen aber die Kurven (und damit der Polymergehalt) mit der gleichen Steigung an wie bei der Kurve des Blindtests. Nach vier Stunden ist so eine Peakfläche von 1400 - 1700 errreicht.

### Beispiel 4 (nicht erfindungsgemäßes Vergleichsbeispiel): TBC

Potentester der bereits in der Literatur beschriebenen Inhibitoren. Die Polymerisation wird im Vergleich zum Blindwert bzw. den TEMPO-Derivaten oder DNBP stark verlangsamt. Auch nach vier Stunden wird erst eine Peakfläche von 640 erreicht.

### Beispiel 5 (nicht erfindungsgemäßes Vergleichsbeispiel): DNBP

Bei "normalen", zur Polymerisation neigenden vinylhaltigen Monomeren, wie z. B. Styrol wirkt DNBP als guter Retarder (siehe nicht erfindungsgemäßes Beispiel 16). Auch wird ihm nachgesagt, dass es in den Diels-Alder-Mechanismus eingreift. Daher wäre zu erwarten, dass DNBP in diesem Test gut abschneidet. Bei Einsatz im Test zeigt sich jedoch, dass DNBP keine Wirkung im Bezug auf die Polymerisation von Dicyclopentadien oder Cyclopentadien hat. Die Polymerisation verläuft in Anwesenheit von DNBP genau gleich wie ohne jeglichen Zusatz. Die detektierte Peakfläche nach zwei bzw. vier Stunden entspricht dem des Blindwerts (Beispiel 1)

### Beispiel 6 (nicht erfindungsgemäßes Vergleichsbeispiel): MeHQ

In manchen in der Literatur beschriebenen Verfahren wird MeHQ als Stabilisator eingesetzt. Im Test mit (Di-)Cyclopentadien zeigt es aber keine Wirkung.

### Beispiele 7 - 11 (erfindungsgemäße Beispiele): Chinonmethide [QM-1, QM-2, QM-5, QM-7, QM-11]

Bei "normalen", zur Polymerisation neigenden vinylhaltigen Monomeren, wie z. B. Styrol, wirken die getesteten Chinonmethide als guter Retarder - genauso gut wie DNBP. Nach Stand der Technik - Vergleich DNBP und Chinonmethide in Styrol - wäre daher zu erwarten, dass die Chinonmethide keine Wirksamkeit zeigen.

Umso erstaunlicher ist es, dass die eingesetzen Chinonmethide, die Polymerisation des Ccyclopentadiens bzw. Dicyclopentadiens sehr stark verlangsamen. Der Effekt ist größer als mit jedem anderen der Inhibitoren, die getestet worden sind.

### Vergleichsversuch unter Nutzung von Styrol als Monomer; Beispiele 12 - 21:

Käuflich erwerbbares, stabilisiertes Styrol wird bei einem reduziertem Druck von 95 mbar und einer Sumpftemperatur von 75 °C in einer inerten Stickstoffatmosphäre von dem Stabilisator *tert*-Butyl-1,2-hydroxybenzol (TBC) befreit. Die Versuchsapparatur, die aus einem Mehrhalskolben, der mit einem Thermometer, einem Rückflusskühler, einem Septum und einem KPG-Rührer versehen ist, wird gründlich mit Stickstoff gespült, um eine sauerstofffreie Atmosphäre zu erhalten. 300 g des unstabilisierten Styrols werden in den Mehrhalskolben gegeben und mit 100 ppm eines Inhibitors gemäß Tabelle 2 versetzt. Durch die ständige Stickstoffzufuhr über eine Glasfritte in die Styrol-Lösung wird eine inerte Stickstoffatmosphäre über die gesamte Versuchsdauer gewährleistet. Die Styrol-Lösung wird kräftig gerührt.

Zum Start des Experiments wird der Kolben in ein auf 110 °C vorgeheiztes Ölbad so weit eingetaucht, dass die stabilisierte Styrol-Lösung komplett eingetaucht ist. Nach dem Eintauchen des Dreihalskolbens in das geheizte Ölbad werden in regelmäßigen Abständen ca. 3 g der Styrol-Lösung über das Septum entnommen, genau ausgewogen und in 50 ml Methanol gegeben. Die Methanol-Mischung wird für eine halbe Stunde bei Raumtemperatur gerührt. Das Methanol bewirkt die Ausfällung des während des Versuchs gebildeten Polystyrols. Dieses wird durch Filtration über einen Glasfiltertiegel abgetrennt. Der Filterrückstand wird mit 20 ml Methanol gewaschen und anschließend für mindestens 5 Stunden bei 110 °C getrocknet. Das im Glasfiltertiegel zurückgebliebene Polystyrol wird nun ausgewogen. Aus dem ermittelten Wert und der Einwaage wird der prozentuale Anteil an Polymer ermittelt. Dieser Polymergehalt wird gegen die Reaktionszeit aufgetragen (vgl. auch weitere Werte, die in Abbildung 2 dargestellt sind)

**Tabelle 2**

| **Beispiel** | **Name und Struktur des Inhibitors** | **Polymergehalt in %** | |
|---|---|---|---|
| | | **nach 120 min** | **nach 210 min** |
| **12** | Kein Inhibitor (Blindprobe) | 9.3 | 16.4 |
| **13** | 4-Hydroxy-TEMPO (4-HT) | 3.0 | 8.2 |
| **14** | 4-Butoxy-TEMPO | 2.4 | 8.1 |
| **15** | TBC | 6.0 | 14.2 |
| **16** | DNBP | 1.2 | 2.8 |
| **17** | **QM-1** | 0.9 | 10.3 |
| **18** | **QM-2** | 1.0 | 2.9 |
| **19** | **QM-5** | 1.1 | 2.8 |
| **20** | **QM-7** | 2.0 | 3.4 |
| **21** | **QM-8** | 2.7 | 5.0 |

### Auswertung der Beispiele 12 - 21

Aus der Tabelle ist ersichtlich, dass die TEMPO-Derivate (Beispiele 13 und 14) die Polymerisation des Styrols kurzfristig gut inhibieren. Danach sind sie aufgebraucht und zeigen nahezu keine Wirkung mehr. Entsprechende Ergebnisse findet man auch im (Di-)Cyclopentadien (Beispiele 2 und 3).

TBC (Beispiel 15) zeigt dagegen im Styroltest nahezu keine Wirkung, während es in (Di-)Cyclopentadien eine recht gute Wirksamkeit aufweist (Beispiel 4).

Bei DNBP (Beispiel 16) ist der Effekt genau umgekehrt. Während diese eine nahezu gleich gute oder sogar bessere Wirksamkeit wie die Chinonmethide **QM-2, QM-5, QM-7, QM-8** (Beispiele 18 - 21) in Styrol aufweist, zeigt DNBP in (Di-)Cyclopentadien keine Wirkung (Beispiel 5). Dagegen sind sämtliche Chinonmethide in (Di-)Cyclopentadien sehr aktiv (Beispiele 7 - 11).

Im Gegensatz zu den übrigen Chinonmethoiden zeigt sich **QM-1** in Styrol als potenter Inhibitor, verliert aber nach kurzer Zeit seine Wirkung (Beispiele 17). Dagegen zeigt es in (Di-)Cyclopentadien erstaunlicherweise eine sehr gute, langanhaltende Wirksamkeit.

### (Beispiel 7)

Aus dem Vergleich der Testergebnisse mit (Di-)Cyclopentadien und Styrol ist zu schließen, dass es sich bei der Polymerisation der beiden doppelbindungshaltigen Monomeren um unterschiedliche Mechanismen handelt. Man kann die Wirksamkeit der Inhibitoren nicht von einem zum anderen Monomer vorhersagen.

In Abbildung 2 sind zum Vergleich die mit **QM-1** und **QM-5** erhaltenen Werte gezeigt.

### Allgemeine Beschreibung - Einsatz in der Cyclopentadien-Herstellung; Beispiele 22 und 23

Ein 500 ml Mehrhalskolben wird ausgelitert und eine Markierung bei 400 mL angebracht. Es ist eine kontinuierliche Zudosierung von frischen Dicyclopentadien vorgesehen. Der Kolben wird mit einer beheizbaren Kolonne, die mit Glasraschigringen bestückt ist, versehen. Das Ölbad wird auf 180 °C temperiert.

Bei den kontinuierlichen Versuchen wird technisches DCPD (93 %) eingesetzt.

Das gesamte einzusetzende Dicyclopentadien wird mit 5000 ppm des zu testenden Inhibitor versetzt. Es werden 100 g Dicyclopentadien (mit Inhibitor) kontinuierlich mit einem leichten Stickstoffstrom überströmt.

Dann wird der Mehrhalskolben ins vortemperierte Ölbad getaucht. Mit dem Erreichen einer Sumpftemperatur von 160°C werden pro Stunde kontinuierlich 30 ml Dicyclopentadien (mit Inhibitor) in den Sumpf zudosiert. Ab einer Temperatur von ca. 164°C beginnt der Sumpf zu sieden, das Dicyclopentadien wird zu Cyclopentadien aufgespalten und über die Kolonne destilliert.

Das hergestellte Cyclopentadien wird in einer auf -3°C gekühlte Vorlage aufgefangen.

Entsteht unter den gegebenen Temperaturen nicht mehr genug Cyclopentadien, steigt der Sumpfstand an. Bei Erreichen eines Sumpfstands von 400 mL wird die Zudosierung beendet und übriges entstandenes Dicyclopentadien und Cyclopentadien aus dem Sumpf destilliert. Die Ergebnisse sind in Tabelle 3 dargestellt.

**Tabelle 3**

| Beispiel | Inhibitor | Laufzeit in h | Gesamtmenge DCPD in g | Isoliertes CPD in g | Ausbeute in % |
|---|---|---|---|---|---|
| **22** | Kein Zusatz | 38.00 | 1197.2 | 854.8 | 76.8 |
| **23** | **QM-5** | 43.50 | 1324 | 976.8 | 79.3 |

Wie der Tabelle zu entnehmen ist, zeigt der Zusatz von **QM-5** einen deutlich verlängernden Effekt auf die Laufzeit der Herstellung von Dicyclopentadien und Cyclopentadien. Anstelle von 38 h kann die Apparatur ohne Sumpfaustausch für 43.5 h betrieben werden. Zudem konnte die Ausbeute an Cyclopentadien bezogen auf den gesamten Einsatz von Dicyclopentadien von 76.8 % auf 79.3 % gesteigert werden.

## Patentansprüche

1. Verfahren zur Inhibierung der Polymerisation von (B), **dadurch gekennzeichnet, dass** (A) und (B) in Kontakt gebracht werden,
wobei
(A) mindestens eine Verbindung der Struktur (I) 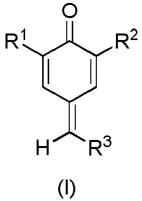 mit
R¹ und R² sind unabhängig voneinander Wasserstoff, Alkylgruppe mit 1 bis 18 Kohlenstoffatomen, Cycloalkylgruppe mit 3 bis 15 Kohlenstoffatomen, Arylgruppe mit 6 bis 15 Kohlenstoffatomen oder Phenylalkylgruppe mit 7 bis 15 Kohlenstoffatomen;
R³ = -CN, -COOH, -COOR⁴, -COR⁵, -OCOR⁶, -CONR⁷R⁸, -PO(OR⁹)₂, -O-R¹⁰ ,-S-R¹¹, -R¹², -C≡C-R¹³ oder Halogen;
mit
R⁴, R⁵, R⁶ = Alkylgruppe mit 1 bis 18 Kohlenstoffatomen, Cycloalkylgruppe mit 3 bis 12 Kohlenstoffatomen, Arylgruppe mit 6 bis 10 Kohlenstoffatomen;
R⁷ und R⁸ sind unabhängig voneinander
Wasserstoff;
Alkylgruppe mit 1 bis 15 Kohlenstoffatomen, die substituiert sein kann mit mindestens einem Substituenten ausgewählt aus der Gruppe bestehend aus Alkylaminogruppe mit 1 bis 4 Kohlenstoffatomen, Dialkylaminogruppe mit 2 bis 8 Kohlenstoffatomen, Hydroxygruppe;
Phenylalkylgruppe mit 7 bis 15 Kohlenstoffatomen, die substituiert sein kann mit mindestens einem Substituenten ausgewählt aus der Gruppe bestehend aus Hydroxygruppe, Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen;
Arylgruppe mit 6 bis 10 Kohlenstoffatomen, die substituiert sein kann mit mindestens einem Substituenten ausgewählt aus der Gruppe bestehend aus Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen, Hydroxygruppe;
oder NR⁷R⁸ ist Morpholino, Piperidino oder Pyrrolidino;
R⁹, R¹⁰, R¹¹ =
Wasserstoff;
Alkylgruppe mit 1 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
Cycloalkylgruppe mit 3 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
Phenylalkylgruppe mit 7 bis 15 Kohlenstoffatomen die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
oder
Arylgruppe mit 6 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
R¹² = 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen; wobei die Reste 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
R¹³ = Wasserstoff, Alkylgruppe mit 1 bis 12 Kohlenstoffatomen, Arylgruppe mit 6 bis 10 Kohlenstoffatomen, wobei die Arylgruppe mit 6 bis 10 Kohlenstoffatomen substituiert sein kann mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
wobei die Substituenten vom Typ R¹, R², R³ gleich oder unterschiedlich sind,
ist,
und
(B) mindestens eine Cyclopentadienverbindung
ist.

2. Verfahren nach Anspruch 1, **dadurch gekennzeichnet, dass** R¹ und R² in der Verbindung der Struktur (I) *tert*-Butyl sind und
R³ = -CN, -COOH, -O-R¹⁰, -S-R¹¹, -R¹² oder -C≡C-R¹³,
mit
R¹⁰ = Methyl, Ethyl, *iso*-Propyl, *n*-Propyl, *n*-Butyl, *iso*-Butyl, sec-Butyl, tert-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl;
R¹¹ = Alkylgruppe mit 1 bis 6 Kohlenstoffatomen;
R¹² = 2-Furylgruppe, 3-Furylgruppe oder Phenyl, wobei Phenyl substituiert sein kann mit mindestens einem Substituenten ausgewählt aus der Gruppe bestehend aus Hydroxygruppe, Alkylgruppe mit 1 bis 8 Kohlenstoffatomen;
R¹³ = Phenyl.

3. Verfahren nach Anspruch 1 oder 2, **dadurch gekennzeichnet, dass** R¹ und R² in der Verbindung der Struktur (I) *tert*-Butyl sind und
R³ = -O-R¹⁰ mit R¹⁰ = Methyl, Ethyl, *n*-Propyl, *iso*-Propyl, *n*-Butyl, *n*-Pentyl oder *n*-Hexyl.

4. Verfahren nach Anspruch 1 oder 2, **dadurch gekennzeichnet, dass** R¹ und R² in der Verbindung der Struktur (I) *tert*-Butyl sind und
R³ = -R¹² mit R¹² = 2-Furylgruppe, 3-Furylgruppe oder Phenyl, bevorzugt 2-Furylgruppe.

5. Verfahren nach einem oder mehreren der Ansprüche 1 bis 4, **dadurch gekennzeichnet, dass** die Cyclopentadienverbindung ausgewählt ist aus der Gruppe bestehend aus Cyclopentadien, Dicyclopentadien, alkyliertes Cyclopentadien und alkyliertes Dicyclopentadien.

6. Verfahren nach einem oder mehreren der Ansprüche 1 bis 5, **dadurch gekennzeichnet, dass** die Cyclopentadienverbindung ausgewählt ist aus der Gruppe bestehend aus Cyclopentadien und Dicyclopentadien.

7. Verfahren nach einem oder mehreren der Ansprüche 1 bis 6, **dadurch gekennzeichnet, dass** (A) als Lösung (AC) in (C) vorliegt, wobei (C) mindestens ein Lösungsmittel ist, wobei das Lösungsmittel bevorzugt ausgewählt ist aus der Gruppe bestehend aus Benzol, einfach oder mehrfach alkylierten Aromaten, Alkanen mit einer Kohlenstoffzahl von 6 bis 15, Cycloalkanen mit einer Kohlenstoffzahl von 6 bis 15, hochsiedenden Kohlenwasserstoffschnitten, Ethern mit einer Kohlenstoffzahl von 6 bis 15 und Estern mit einer Kohlenstoffzahl von 6 bis 15.

8. Verfahren nach Anspruch 7, **dadurch gekennzeichnet, dass** das Verhältnis (m/m) des Gesamtgewichts aller Verbindungen der Struktur (I) in der Lösung (AC) zum Gesamtgewicht aller Lösungsmittel in der Lösung (AC) im Bereich von 1 : 1000 bis 100 : 1 liegt.

9. Verfahren nach einem oder mehreren der Ansprüche 1 bis 8, **dadurch gekennzeichnet, dass** die Gesamtkonzentration aller Verbindungen der Struktur (I) zwischen 10 ppb (m/m) und 100000 ppm (m/m), bezogen auf das Gesamtgewicht aller Cyclopentadienverbindungen, beträgt.

10. Verfahren nach einem oder mehreren der Ansprüche 1 bis 9, **dadurch gekennzeichnet, dass** (B) in einem Prozessstrom in einer Konzentration von 0.0001 bis 15 Gew.-% vorliegt.

11. Zusammensetzung (AB), umfassend (A) und (B), wobei
(A) mindestens eine Verbindung der Struktur (I) 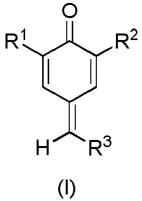 mit
R¹ und R² sind unabhängig voneinander Wasserstoff, Alkylgruppe mit 1 bis 18 Kohlenstoffatomen, Cycloalkylgruppe mit 3 bis 15 Kohlenstoffatomen, Arylgruppe mit 6 bis 15 Kohlenstoffatomen oder Phenylalkylgruppe mit 7 bis 15 Kohlenstoffatomen;
R³ = -CN, -COOH, -COOR⁴, -COR⁵, -OCOR⁶, -CONR⁷R⁸, -PO(OR⁹)₂, -O-R¹⁰, -S-R¹¹, -R¹², -C≡C-R¹³ oder Halogen;
mit
R⁴, R⁵, R⁶ = Alkylgruppe mit 1 bis 18 Kohlenstoffatomen, Cycloalkylgruppe mit 3 bis 12 Kohlenstoffatomen, Arylgruppe mit 6 bis 10 Kohlenstoffatomen;
R⁷ und R⁸ sind unabhängig voneinander
Wasserstoff;
Alkylgruppe mit 1 bis 15 Kohlenstoffatomen, die substituiert sein kann mit mindestens einem Substituenten ausgewählt aus der Gruppe bestehend aus Alkylaminogruppe mit 1 bis 4 Kohlenstoffatomen, Dialkylaminogruppe mit 2 bis 8 Kohlenstoffatomen, Hydroxygruppe;
Phenylalkylgruppe mit 7 bis 15 Kohlenstoffatomen, die substituiert sein kann mit mindestens einem Substituenten ausgewählt aus der Gruppe bestehend aus Hydroxygruppe, Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen;
Arylgruppe mit 6 bis 10 Kohlenstoffatomen, die substituiert sein kann mit mindestens einem Substituenten ausgewählt aus der Gruppe bestehend aus Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen, Hydroxygruppe;
oder NR⁷R⁸ ist Morpholino, Piperidino oder Pyrrolidino;
R⁹, R¹⁰, R¹¹ =
Wasserstoff;
Alkylgruppe mit 1 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
Cycloalkylgruppe mit 3 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
Phenylalkylgruppe mit 7 bis 15 Kohlenstoffatomen die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
oder
Arylgruppe mit 6 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
R¹² = 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen; wobei die Reste 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
R¹³ = Wasserstoff, Alkylgruppe mit 1 bis 12 Kohlenstoffatomen, Arylgruppe mit 6 bis 10 Kohlenstoffatomen, wobei die Arylgruppe mit 6 bis 10 Kohlenstoffatomen substituiert sein kann mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
wobei die Substituenten vom Typ R¹, R², R³ gleich oder unterschiedlich sind, ist,
und
(B) mindestens eine Cyclopentadienverbindung
ist.

12. Zusammensetzung (AB) nach Anspruch 11, **dadurch gekennzeichnet, dass** R¹ und R² in der Verbindung der Struktur (I) *tert*-Butyl sind und
R³ = -CN, -COOH, -O-R¹⁰, -S-R¹¹, -R¹² oder -C≡C-R¹³;
mit
R¹⁰ = Methyl, Ethyl, *iso*-Propyl, *n*-Propyl, *n*-Butyl, *iso*-Butyl, sec-Butyl, tert-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl;
R¹¹ = Alkylgruppe mit 1 bis 6 Kohlenstoffatomen;
R¹² = 2-Furylgruppe, 3-Furylgruppe oder Phenyl, wobei Phenyl substituiert sein kann mit mindestens einem Substituenten ausgewählt aus der Gruppe bestehend aus Hydroxygruppe, Alkylgruppe mit 1 bis 8 Kohlenstoffatomen;
R¹³ = Phenyl.

13. Zusammensetzung (AB) nach Anspruch 11 oder 12, **dadurch gekennzeichnet, dass** R¹ und R² in der Verbindung der Struktur (I) *tert*-Butyl sind und
R³ = -O-R¹⁰ mit R¹⁰ = Methyl, Ethyl, *n*-Propyl, *iso*-Propyl, *n*-Butyl, *n*-Pentyl oder *n*-Hexyl.

14. Zusammensetzung (AB) nach Anspruch 11 oder 12, **dadurch gekennzeichnet, dass** R¹ und R² in der Verbindung der Struktur (I) *tert*-Butyl sind und
R³ = -R¹² mit R¹² = 2-Furylgruppe, 3-Furylgruppe oder Phenyl, bevorzugt 2-Furylgruppe.

15. Zusammensetzung (AB) nach einem oder mehreren der Ansprüche 11 bis 14, **dadurch gekennzeichnet, dass** die Cyclopentadienverbindung ausgewählt ist aus der Gruppe bestehend aus Cyclopentadien, Dicyclopentadien, alkyliertes Cyclopentadien und alkyliertes Dicyclopentadien.

16. Zusammensetzung (AB) nach einem oder mehreren der Ansprüche 11 bis 15, **dadurch gekennzeichnet, dass** die Cyclopentadienverbindung ausgewählt ist aus der Gruppe bestehend aus Cyclopentadien und Dicyclopentadien.

17. Zusammensetzung (AB) nach einem oder mehreren der Ansprüche 11 bis 16, **dadurch gekennzeichnet, dass** die Gesamtkonzentration aller Verbindungen der Struktur (I) in der Zusammensetzung (AB) zwischen 10 ppb (m/m) und 100000 ppm (m/m), bezogen auf das Gesamtgewicht aller Cyclopentadienverbindungen in der Zusammensetzung (AB) beträgt.

18. Verwendung von (A) zur Inhibierung der Polymerisation von (B),
wobei
(A) mindestens eine Verbindung der Struktur (I) 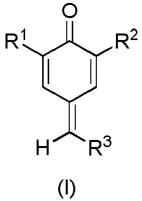 mit
R¹ und R² sind unabhängig voneinander Wasserstoff, Alkylgruppe mit 1 bis 18 Kohlenstoffatomen, Cycloalkylgruppe mit 3 bis 15 Kohlenstoffatomen, Arylgruppe mit 6 bis 15 Kohlenstoffatomen oder Phenylalkylgruppe mit 7 bis 15 Kohlenstoffatomen;
R³ = -CN, -COOH, -COOR⁴, -COR⁵, -OCOR⁶, -CONR⁷R⁸, -PO(OR⁹)₂, -O-R¹⁰,-S-R¹¹, -R¹², -C≡C-R¹³ oder Halogen;
mit
R⁴, R⁵, R⁶ = Alkylgruppe mit 1 bis 18 Kohlenstoffatomen, Cycloalkylgruppe mit 3 bis 12 Kohlenstoffatomen, Arylgruppe mit 6 bis 10 Kohlenstoffatomen;
R⁷ und R⁸ sind unabhängig voneinander
Wasserstoff;
Alkylgruppe mit 1 bis 15 Kohlenstoffatomen, die substituiert sein kann mit mindestens einem Substituenten ausgewählt aus der Gruppe bestehend aus Alkylaminogruppe mit 1 bis 4 Kohlenstoffatomen, Dialkylaminogruppe mit 2 bis 8 Kohlenstoffatomen, Hydroxygruppe;
Phenylalkylgruppe mit 7 bis 15 Kohlenstoffatomen, die substituiert sein kann mit mindestens einem Substituenten ausgewählt aus der Gruppe bestehend aus Hydroxygruppe, Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen;
Arylgruppe mit 6 bis 10 Kohlenstoffatomen, die substituiert sein kann mit mindestens einem Substituenten ausgewählt aus der Gruppe bestehend aus Alkylgruppe mit 1 bis 4 Kohlenstoffatomen, Alkylaminogruppe mit 1 bis 4 Kohlenstoffatomen, Dialkylaminogruppe mit 2 bis 8 Kohlenstoffatomen, Hydroxygruppe;
oder NR⁷R⁸ ist Morpholino, Piperidino oder Pyrrolidino;
R⁹, R¹⁰, R¹¹ =
Wasserstoff;
Alkylgruppe mit 1 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴,
-[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
Cycloalkylgruppe mit 3 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
Phenylalkylgruppe mit 7 bis 15 Kohlenstoffatomen die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen; oder
Arylgruppe mit 6 bis 15 Kohlenstoffatomen, die substituiert sein kann mit mindestens einem Substituenten ausgewählt aus der Gruppe bestehend aus Hydroxygruppe, Dialkylaminogruppe, -OR¹⁴, -[O(CH₂)_{y}]ₓH, wobei x = 1, 2, 3, 4, 5, 6, 7, 8, 9 oder 10 und y = 1, 2, 3 oder 4 und wobei R¹⁴ = Alkylgruppe mit 1 bis 6 Kohlenstoffatomen;
R¹² = 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen; wobei die Reste 2-Pyridylgruppe, 3-Pyridylgruppe, 4-Pyridylgruppe, 2-Thienylgruppe, 3-Thienylgruppe, 2-Pyrrylgruppe, 3-Pyrrylgruppe, 2-Furylgruppe, 3-Furylgruppe oder Arylgruppe mit 6 bis 15 Kohlenstoffatomen substituiert sein können mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
R¹³ = Wasserstoff, Alkylgruppe mit 1 bis 12 Kohlenstoffatomen, Arylgruppe mit 6 bis 10 Kohlenstoffatomen, wobei die Arylgruppe mit 6 bis 10 Kohlenstoffatomen substituiert sein kann mit mindestens einem Substituenten ausgewählt aus der Gruppe bestehend aus Hydroxygruppe, Nitrogruppe, Aminogruppe, Cyanogruppe, Carboxygruppe, Aminocarbonylgruppe, Halogen, Alkylgruppe mit 1 bis 8 Kohlenstoffatomen, Alkoxygruppe mit 1 bis 8 Kohlenstoffatomen, Alkylthiogruppe mit 1 bis 8 Kohlenstoffatomen, Alkylaminogruppe mit 1 bis 8 Kohlenstoffatomen, Dialkylaminogruppe mit 2 bis 8 Kohlenstoffatomen und Carbonsäureestergruppe mit 2 bis 8 Kohlenstoffatomen;
wobei die Substituenten vom Typ R¹, R², R³ gleich oder unterschiedlich sind, ist,
und
(B) mindestens eine Cyclopentadienverbindung
ist.

19. Verwendung nach Anspruch 18, **dadurch gekennzeichnet, dass** R¹ und R² in der Verbindung der Struktur (I) *tert*-Butyl sind und
R³ = -CN, -COOH, -O-R¹⁰, -S-R¹¹, -R¹² oder -C≡C-R¹³; mit
R¹⁰ = Methyl, Ethyl, *iso*-Propyl, *n*-Propyl, *n*-Butyl, *iso*-Butyl, sec-Butyl, tert-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethypropyl, 1,2,2-Trimethylpropyl, 1-Ethyl-1-methylpropyl oder 1-Ethyl-2-methylpropyl; R¹¹ = Alkylgruppe mit 1 bis 6 Kohlenstoffatomen;
R¹² = 2-Furylgruppe, 3-Furylgruppe oder Phenyl, wobei Phenyl substituiert sein kann mit mindestens einem Substituenten ausgewählt aus der Gruppe bestehend aus Hydroxygruppe, Alkylgruppe mit 1 bis 8 Kohlenstoffatomen;
R¹³ = Phenyl.

20. Verwendung nach Anspruch 18 oder 19, **dadurch gekennzeichnet, dass** R¹ und R² in der Verbindung der Struktur (I) *tert*-Butyl sind und
R³ = -O-R¹⁰ mit R¹⁰ = Methyl, Ethyl, *n*-Propyl, *iso*-Propyl, *n*-Butyl, *n*-Pentyl oder *n*-Hexyl.

21. Verwendung nach Anspruch 18 oder 19, **dadurch gekennzeichnet, dass** R¹ und R² in der Verbindung der Struktur (I) *tert*-Butyl sind und
R³ = -R¹² mit R¹² = 2-Furylgruppe, 3-Furylgruppe oder Phenyl, bevorzugt 2-Furylgruppe.

22. Verwendung nach einem oder mehreren der Ansprüche 18 bis 21, **dadurch gekennzeichnet, dass** die Cyclopentadienverbindung ausgewählt ist aus der Gruppe bestehend aus Cyclopentadien, Dicyclopentadien, alkyliertes Cyclopentadien und alkyliertes Dicyclopentadien.

23. Verwendung nach einem oder mehreren der Ansprüche 18 bis 22, **dadurch gekennzeichnet, dass** die Cyclopentadienverbindung ausgewählt ist aus der Gruppe bestehend aus Cyclopentadien und Dicyclopentadien.

## Claims

1. Process for inhibiting the polymerization of (B), **characterized in that** (A) and (B) are brought into contact,
wherein
(A) is at least one compound of structure (I) 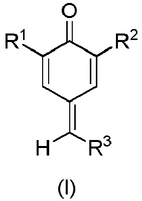 where
R¹ and R² are each independently hydrogen, alkyl of 1 to 18 carbon atoms, cycloalkyl of 3 to 15 carbon atoms, aryl of 6 to 15 carbon atoms or phenylalkyl of 7 to 15 carbon atoms;
R³ = -CN, -COOH, -COOR⁴, -COR⁵, -OCOR⁶, -CONR⁷R⁸, -PO(OR⁹)₂, -O-R¹⁰, -S-R¹¹, -R¹², -C≡C-R¹³ or halogen; where
R⁴, R⁵, R⁶ = alkyl of 1 to 18 carbon atoms, cycloalkyl of 3 to 12 carbon atoms, aryl of 6 to 10 carbon atoms;
R⁷ and R⁸ are each independently
hydrogen;
alkyl of 1 to 15 carbon atoms which is unsubstituted or substituted with at least one substituent selected from the group consisting of alkylamino having 1 to 4 carbon atoms, dialkylamino having 2 to 8 carbon atoms and hydroxyl;
phenylalkyl of 7 to 15 carbon atoms which is unsubstituted or substituted with at least one substituent selected from the group consisting of hydroxyl, alkyl having 1 to 4 carbon atoms, alkylamino having 1 to 4 carbon atoms and dialkylamino having 2 to 8 carbon atoms;
aryl of 6 to 10 carbon atoms which is unsubstituted or substituted with at least one substituent selected from the group consisting of alkyl having 1 to 4 carbon atoms, alkylamino having 1 to 4 carbon atoms, dialkylamino having 2 to 8 carbon atoms and hydroxyl;
or NR⁷R⁸ is morpholino, piperidino or pyrrolidino;
R⁹, R¹⁰, R¹¹ =
hydrogen;
alkyl of 1 to 15 carbon atoms which is unsubstituted or substituted with at least one substituent selected from the group consisting of hydroxyl, dialkylamino,-OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
cycloalkyl of 3 to 15 carbon atoms which is unsubstituted or substituted with at least one substituent selected from the group consisting of hydroxyl, dialkylamino, - OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
phenylalkyl of 7 to 15 carbon atoms which is unsubstituted or substituted with at least one substituent selected from the group consisting of hydroxyl, dialkylamino, -OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms; or
aryl of 6 to 15 carbon atoms which is unsubstituted or substituted with at least one substituent selected from the group consisting of hydroxyl, dialkylamino,-OR¹⁴, - [O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
R¹² = 2-pyridyl, 3-pyridyl, 4-pyridyl, 2-thienyl, 3-thienyl, 2-pyrryl, 3-pyrryl, 2-furyl, 3-furyl or aryl of 6 to 15 carbon atoms; wherein the radicals 2-pyridyl, 3-pyridyl, 4-pyridyl, 2-thienyl, 3-thienyl, 2-pyrryl, 3-pyrryl, 2-furyl, 3-furyl or aryl of 6 to 15 carbon atoms are unsubstituted or substituted with at least one substituent selected from the group consisting of hydroxyl, nitro, amino, cyano, carboxyl, aminocarbonyl, halogen, alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms and a carboxylic ester group of 2 to 8 carbon atoms;
R¹³= hydrogen, alkyl of 1 to 12 carbon atoms, aryl of 6 to 10 carbon atoms, wherein the aryl of 6 to 10 carbon atoms is unsubstituted or substituted with at least one substituent selected from the group consisting of hydroxyl, nitro, amino, cyano, carboxyl, aminocarbonyl, halogen, alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms and a carboxylic ester group of 2 to 8 carbon atoms;
wherein the substituents R¹, R² and R³ are the same or different;
and
(B) is at least one cyclopentadiene compound.

2. Process according to Claim 1, **characterized in that** R¹ and R² are each *tert*-butyl in the compound of structure (I) and R³ = -CN, -COOH, -O-R¹⁰, -S-R¹¹, -R¹² or -C≡C-R¹³;
where
R¹⁰ = methyl, ethyl, *iso*-propyl, *n*-propyl, *n*-butyl, *iso-*butyl, sec-butyl, tert-butyl, *n*-pentyl, 1-methylbutyl, 2-methylbutyl, 3-methylbutyl, 1,1-dimethylpropyl, 1,2-dimethylpropyl, 2,2-dimethylpropyl, 1-ethylpropyl, *n*-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl;
R¹¹ = alkyl of 1 to 6 carbon atoms;
R¹² = 2-furyl, 3-furyl or phenyl, wherein phenyl is unsubstituted or substituted with at least one substituent selected from the group consisting of hydroxyl and alkyl of 1 to 8 carbon atoms;
R¹³ = phenyl.

3. Process according to Claim 1 or 2, **characterized in that** R¹ and R² are each *tert*-butyl in the compound of structure (I) and
R³ = -O-R¹⁰ where R¹⁰ = methyl, ethyl, *n*-propyl, *iso-*propyl, *n*-butyl, *n*-pentyl or *n*-hexyl.

4. Process according to Claim 1 or 2, **characterized in that** R¹ and R² are each *tert*-butyl in the compound of structure (I) and
R³ = -R¹² where R¹² = 2-furyl, 3-furyl or phenyl, preferably 2-furyl.

5. Process according to one or more of Claims 1 to 4, **characterized in that** the cyclopentadiene compound is selected from the group consisting of cyclopentadiene, dicyclopentadiene, alkylated cyclopentadiene and alkylated dicyclopentadiene.

6. Process according to one or more of Claims 1 to 5, **characterized in that** the cyclopentadiene compound is selected from the group consisting of cyclopentadiene and dicyclopentadiene.

7. Process according to one or more of Claims 1 to 6, **characterized in that** (A) is in the form of a solution (AC) in (C), wherein (C) is at least one solvent, wherein the solvent is preferably selected from the group consisting of benzene, mono- or polyalkylated aromatics, alkanes having a carbon number of 6 to 15, cycloalkanes having a carbon number of 6 to 15, high-boiling hydrocarbon cuts, ethers having a carbon number of 6 to 15 and esters having a carbon number of 6 to 15.

8. Process according to Claim 7, **characterized in that** the total weight of all compounds of structure (I) in solution (AC) has an (m/m) ratio to the total weight of all solvents in said solution (AC) in the range from 1:1000 to 100:1.

9. Process according to one or more of Claims 1 to 8, **characterized in that** the total concentration of all compounds of structure (I) is between 10 ppb (m/m) and 100 000 ppm (m/m), based on the total weight of all cyclopentadiene compounds.

10. Process according to one or more of Claims 1 to 9, **characterized in that** (B) is in a process stream at a concentration of 0.0001 to 15 wt%.

11. Composition (AB), comprising (A) and (B),
wherein
(A) is at least one compound of structure (I) 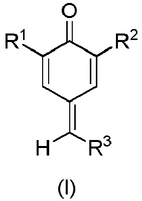 where
R¹ and R² are each independently hydrogen, alkyl of 1 to 18 carbon atoms, cycloalkyl of 3 to 15 carbon atoms, aryl of 6 to 15 carbon atoms or phenylalkyl of 7 to 15 carbon atoms;
R³ = -CN, -COOH, -COOR⁴, -COR⁵, -OCOR⁶, -CONR⁷R⁸, -PO(OR⁹)₂, -O-R¹⁰, -S-R¹¹, -R¹², -C≡C-R¹³ or halogen;
where
R⁴, R⁵, R⁶ = alkyl of 1 to 18 carbon atoms, cycloalkyl of 3 to 12 carbon atoms, aryl of 6 to 10 carbon atoms;
R⁷ and R⁸ are each independently
hydrogen;
alkyl of 1 to 15 carbon atoms which is unsubstituted or substituted with at least one substituent selected from the group consisting of alkylamino having 1 to 4 carbon atoms, dialkylamino having 2 to 8 carbon atoms and hydroxyl;
phenylalkyl of 7 to 15 carbon atoms which is unsubstituted or substituted with at least one substituent selected from the group consisting of hydroxyl, alkyl having 1 to 4 carbon atoms, alkylamino having 1 to 4 carbon atoms and dialkylamino having 2 to 8 carbon atoms;
aryl of 6 to 10 carbon atoms which is unsubstituted or substituted with at least one substituent selected from the group consisting of alkyl having 1 to 4 carbon atoms, alkylamino having 1 to 4 carbon atoms, dialkylamino having 2 to 8 carbon atoms and hydroxyl;
or NR⁷R⁸ is morpholino, piperidino or pyrrolidino;
R⁹, R¹⁰, R¹¹ =
hydrogen;
alkyl of 1 to 15 carbon atoms which is unsubstituted or substituted with at least one substituent selected from the group consisting of hydroxyl, dialkylamino,-OR¹⁴, - [O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
cycloalkyl of 3 to 15 carbon atoms which is unsubstituted or substituted with at least one substituent selected from the group consisting of hydroxyl, dialkylamino, - OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
phenylalkyl of 7 to 15 carbon atoms which is unsubstituted or substituted with at least one substituent selected from the group consisting of hydroxyl, dialkylamino, -OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms; or
aryl of 6 to 15 carbon atoms which is unsubstituted or substituted with at least one substituent selected from the group consisting of hydroxyl, dialkylamino,-OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
R¹² = 2-pyridyl, 3-pyridyl, 4-pyridyl, 2-thienyl, 3-thienyl, 2-pyrryl, 3-pyrryl, 2-furyl, 3-furyl or aryl of 6 to 15 carbon atoms; wherein the radicals 2-pyridyl, 3-pyridyl, 4-pyridyl, 2-thienyl, 3-thienyl, 2-pyrryl, 3-pyrryl, 2-furyl, 3-furyl or aryl of 6 to 15 carbon atoms are unsubstituted or substituted with at least one substituent selected from the group consisting of hydroxyl, nitro, amino, cyano, carboxyl, aminocarbonyl, halogen, alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms and a carboxylic ester group of 2 to 8 carbon atoms;
R¹³ = hydrogen, alkyl of 1 to 12 carbon atoms, aryl of 6 to 10 carbon atoms, wherein the aryl of 6 to 10 carbon atoms is unsubstituted or substituted with at least one substituent selected from the group consisting of hydroxyl, nitro, amino, cyano, carboxyl, aminocarbonyl, halogen, alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms and a carboxylic ester group of 2 to 8 carbon atoms;
wherein the substituents R¹, R² and R³ are the same or different;
and
(B) is at least one cyclopentadiene compound.

12. Composition (AB) according to Claim 11, **characterized in that** R¹ and R² are each *tert*-butyl in the compound of structure (I) and
R³ = -CN, -COOH, -O-R¹⁰, -S-R¹¹, -R¹² or -C≡C-R¹³;
where
R¹⁰ = methyl, ethyl, *iso*-propyl, *n*-propyl, *n*-butyl, *iso-*butyl, sec-butyl, tert-butyl, *n*-pentyl, 1-methylbutyl, 2-methylbutyl, 3-methylbutyl, 1,1-dimethylpropyl, 1,2-dimethylpropyl, 2,2-dimethylpropyl, 1-ethylpropyl, *n*-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl;
R¹¹ = alkyl of 1 to 6 carbon atoms;
R¹² = 2-furyl, 3-furyl or phenyl, wherein phenyl is unsubstituted or substituted with at least one substituent selected from the group consisting of hydroxyl and alkyl of 1 to 8 carbon atoms;
R¹³ = phenyl.

13. Composition (AB) according to Claim 11 or 12, **characterized in that** R¹ and R² are each *tert*-butyl in the compound of structure (I) and
R³ = -O-R¹⁰ where R¹⁰ = methyl, ethyl, *n*-propyl, *iso-*propyl, *n*-butyl, *n*-pentyl or *n*-hexyl.

14. Composition (AB) according to Claim 11 or 12, **characterized in that** R¹ and R² are each *tert*-butyl in the compound of structure (I) and
R³ = -R¹² where R¹² = 2-furyl, 3-furyl or phenyl, preferably 2-furyl.

15. Composition (AB) according to one or more of Claims 11 to 14, **characterized in that** the cyclopentadiene compound is selected from the group consisting of cyclopentadiene, dicyclopentadiene, alkylated cyclopentadiene and alkylated dicyclopentadiene.

16. Composition (AB) according to one or more of Claims 11 to 15, **characterized in that** the cyclopentadiene compound is selected from the group consisting of cyclopentadiene and dicyclopentadiene.

17. Composition (AB) according to one or more of Claims 11 to 16, **characterized in that** the total concentration of all compounds of structure (I) in said composition (AB) is between 10 ppb (m/m) and 100 000 ppm (m/m), based on the total weight of all cyclopentadiene compounds in said composition (AB).

18. Use of (A) for inhibiting the polymerization of (B), wherein
(A) is at least one compound of structure (I) 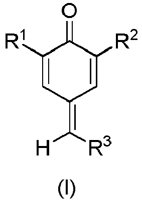 where
R¹ and R² are each independently hydrogen, alkyl of 1 to 18 carbon atoms, cycloalkyl of 3 to 15 carbon atoms, aryl of 6 to 15 carbon atoms or phenylalkyl of 7 to 15 carbon atoms;
R³ = -CN, -COOH, -COOR⁴, -COR⁵, -OCOR⁶, -CONR⁷R⁸, -PO(OR⁹)₂, -O-R¹⁰,-S-R¹¹, -R¹², -C≡C-R¹³ or halogen;
where
R⁴, R⁵, R⁶ = alkyl of 1 to 18 carbon atoms, cycloalkyl of 3 to 12 carbon atoms, aryl of 6 to 10 carbon atoms;
R⁷ and R⁸ are each independently
hydrogen;
alkyl of 1 to 15 carbon atoms which is unsubstituted or substituted with at least one substituent selected from the group consisting of alkylamino having 1 to 4 carbon atoms, dialkylamino having 2 to 8 carbon atoms and hydroxyl;
phenylalkyl of 7 to 15 carbon atoms which is unsubstituted or substituted with at least one substituent selected from the group consisting of hydroxyl, alkyl having 1 to 4 carbon atoms, alkylamino having 1 to 4 carbon atoms and dialkylamino having 2 to 8 carbon atoms;
aryl of 6 to 10 carbon atoms which is unsubstituted or substituted with at least one substituent selected from the group consisting of alkyl having 1 to 4 carbon atoms, alkylamino having 1 to 4 carbon atoms, dialkylamino having 2 to 8 carbon atoms and hydroxyl;
or NR⁷R⁸ is morpholino, piperidino or pyrrolidino;
R⁹, R¹⁰, R¹¹ =
hydrogen;
alkyl of 1 to 15 carbon atoms which is unsubstituted or substituted with at least one substituent selected from the group consisting of hydroxyl, dialkylamino,-OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
cycloalkyl of 3 to 15 carbon atoms which is unsubstituted or substituted with at least one substituent selected from the group consisting of hydroxyl, dialkylamino, - OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
phenylalkyl of 7 to 15 carbon atoms which is unsubstituted or substituted with at least one substituent selected from the group consisting of hydroxyl, dialkylamino, -OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms; or
aryl of 6 to 15 carbon atoms which is unsubstituted or substituted with at least one substituent selected from the group consisting of hydroxyl, dialkylamino,-OR¹⁴, -[O(CH₂)_{y}]ₓH, where x = 1, 2, 3, 4, 5, 6, 7, 8, 9 or 10 and y = 1, 2, 3 or 4 and where R¹⁴ = alkyl of 1 to 6 carbon atoms;
R¹² = 2-pyridyl, 3-pyridyl, 4-pyridyl, 2-thienyl, 3-thienyl, 2-pyrryl, 3-pyrryl, 2-furyl, 3-furyl or aryl of 6 to 15 carbon atoms; wherein the radicals 2-pyridyl, 3-pyridyl, 4-pyridyl, 2-thienyl, 3-thienyl, 2-pyrryl, 3-pyrryl, 2-furyl, 3-furyl or aryl of 6 to 15 carbon atoms are unsubstituted or substituted with at least one substituent selected from the group consisting of hydroxyl, nitro, amino, cyano, carboxyl, aminocarbonyl, halogen, alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms and a carboxylic ester group of 2 to 8 carbon atoms;
R¹³ = hydrogen, alkyl of 1 to 12 carbon atoms, aryl of 6 to 10 carbon atoms, wherein the aryl of 6 to 10 carbon atoms is unsubstituted or substituted with at least one substituent selected from the group consisting of hydroxyl, nitro, amino, cyano, carboxyl, aminocarbonyl, halogen, alkyl of 1 to 8 carbon atoms, alkoxy of 1 to 8 carbon atoms, alkylthio of 1 to 8 carbon atoms, alkylamino of 1 to 8 carbon atoms, dialkylamino of 2 to 8 carbon atoms and a carboxylic ester group of 2 to 8 carbon atoms;
wherein the substituents R¹, R² and R³ are the same or different;
and
(B) is at least one cyclopentadiene compound.

19. Use according to Claim 18, **characterized in that** R¹ and R² are each *tert*-butyl in the compound of structure (I) and
R³ = -CN, -COOH, -O-R¹⁰, -S-R¹¹, -R¹² or -C≡C-R¹³;
where
R¹⁰ = methyl, ethyl, *iso*-propyl, *n*-propyl, *n*-butyl, *iso-*butyl, sec-butyl, tert-butyl, *n*-pentyl, 1-methylbutyl, 2-methylbutyl, 3-methylbutyl, 1,1-dimethylpropyl, 1,2-dimethylpropyl, 2,2-dimethylpropyl, 1-ethylpropyl, *n*-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl;
R¹¹ = alkyl of 1 to 6 carbon atoms;
R¹² = 2-furyl, 3-furyl or phenyl, wherein phenyl is unsubstituted or substituted with at least one substituent selected from the group consisting of hydroxyl and alkyl of 1 to 8 carbon atoms;
R¹³ = phenyl.

20. Use according to Claim 18 or 19, **characterized in that** R¹ and R² are each *tert*-butyl in the compound of structure (I) and
R³ = -O-R¹⁰ where R¹⁰ = methyl, ethyl, *n*-propyl, *iso-*propyl, *n*-butyl, *n*-pentyl or *n*-hexyl.

21. Use according to Claim 18 or 19, **characterized in that** R¹ and R² are each *tert*-butyl in the compound of structure (I) and
R³ = -R¹² where R¹² = 2-furyl, 3-furyl or phenyl, preferably 2-furyl.

22. Use according to one or more of Claims 18 to 21, **characterized in that** the cyclopentadiene compound is selected from the group consisting of cyclopentadiene, dicyclopentadiene, alkylated cyclopentadiene and alkylated dicyclopentadiene.

23. Use according to one or more of Claims 18 to 22, **characterized in that** the cyclopentadiene compound is selected from the group consisting of cyclopentadiene and dicyclopentadiene.

## Revendications

1. Procédé pour inhiber la polymérisation de (B), **caractérisé en ce qu'**on met en contact (A) et (B),
dans lequel
(A) est au moins un composé de structure (I) 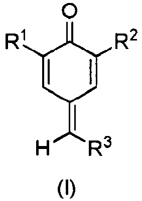 dans laquelle
R¹ et R² représentant chacun indépendamment de l'autre un atome d'hydrogène, un groupe alkyle ayant 1 à 18 atomes de carbone, un groupe cycloalkyle ayant 3 à 15 atomes de carbone, un groupe aryle ayant 6 à 15 atomes de carbone ou un groupe phénylalkyle ayant 7 à 15 atomes de carbone ;
R³ = -CN, -COOH, -COOR⁴, -COR⁵, -OCOR⁶, -CONR⁷R⁸, -PO(OR⁹)₂, -O-R¹⁰, -S-R¹¹, -R¹², -C≡C-R¹³ ou un groupe halogéno ;
où
R⁴, R⁵, R⁶ représentent chacun un groupe alkyle ayant 1 à 18 atomes de carbone, un groupe cycloalkyle ayant 3 à 12 atomes de carbone, un groupe aryle ayant 6 à 10 atomes de carbone ;
R⁷ et R⁸ représentent chacun indépendamment de l'autre : un atome d'hydrogène ;
un groupe alkyle ayant 1 à 15 atomes de carbone, qui peut être substitué par au moins un substituant choisi dans le groupe consistant en les groupes alkylamino ayant 1 à 4 atomes de carbone, les groupes dialkylamino ayant 2 à 8 atomes de carbone, le groupe hydroxy ;
un groupe phénylalkyle ayant 7 à 15 atomes de carbone, qui peut être substitué par au moins un substituant choisi dans le groupe consistant en le groupe hydroxy, les groupes alkyle ayant 1 à 4 atomes de carbone, les groupes alkylamino ayant 1 à 4 atomes de carbone, les groupes dialkylamino ayant 2 à 8 atomes de carbone ;
un groupe aryle ayant 6 à 10 atomes de carbone, qui peut être substitué par au moins un substituant choisi dans le groupe consistant en les groupes alkyle ayant 1 à 4 atomes de carbone, les groupes alkylamino ayant 1 à 4 atomes de carbone, les groupes dialkylamino ayant 2 à 8 atomes de carbone, le groupe hydroxy ;
ou NR⁷R⁸ est le groupe morpholino, pipéridino ou pyrrolidino ;
R⁹, R¹⁰, R¹¹ représentent chacun
un atome d'hydrogène ;
un groupe alkyle ayant 1 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10 et y = 1, 2, 3 ou 4, et R¹⁴ est un groupe alkyle ayant 1 à 6 atomes de carbone ;
un groupe cycloalkyle ayant 3 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10 et y = 1, 2, 3 ou 4, et R¹⁴ est un groupe alkyle ayant 1 à 6 atomes de carbone ;
un groupe phénylalkyle ayant 7 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10, et y = 1, 2, 3 ou 4, et où R¹⁴ est un groupe alkyle ayant 1 à 6 atomes de carbone ;
ou
un groupe aryle ayant 6 à 15 atomes de carbone, qui peut être substitué par un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10, et y = 1, 2, 3 ou 4, et où R¹⁴ est un groupe alkyle ayant 1 à 6 atomes de carbone ;
R¹² est le groupe 2-pyridyle, le groupe 3-pyridyle, le groupe 4-pyridyle, le groupe 2-thiényle, le groupe 3-thiényle, le groupe 2-pyrryle, le groupe 3-pyrryle, le groupe 2-furyle, le groupe 3-furyle ou un groupe aryle ayant 6 à 15 atomes de carbone ; où les radicaux groupe 2-pyridyle, groupe 3-pyridyle, groupe 4-pyridyle, groupe 2-thiényle, groupe 3-thiényle, groupe 2-pyrryle, groupe 3-pyrryle, groupe 2-furyle, groupe 3-furyle ou groupes aryle ayant 6 à 15 atomes de carbone peuvent être substitués par au moins un substituant choisi dans le groupe consistant en le groupe hydroxy, le groupe nitro, le groupe amino, le groupe cyano, le groupe carboxy, le groupe aminocarbonyle, les groupes halogéno, les groupes alkyle ayant 1 à 8 atomes de carbone, les groupes alcoxy ayant 1 à 8 atomes de carbone, les groupes alkylthio ayant 1 à 8 atomes de carbone, les groupes alkylamino ayant 1 à 8 atomes de carbone, les groupes dialkylamino ayant 2 à 8 atomes de carbone et les groupes esters d'acides carboxyliques ayant 2 à 8 atomes de carbone ;
R¹³ est un atome d'hydrogène, un groupe alkyle ayant 1 à 12 atomes de carbone, un groupe aryle ayant 6 à 10 atomes de carbone, où le groupe aryle ayant 6 à 10 atomes de carbone peut être substitué par au moins un substituant choisi dans le groupe consistant en les groupes hydroxy, les groupes nitro, les groupes amino, les groupes cyano, les groupes carboxy, les groupes aminocarbonyle, les groupes halogéno, les groupes alkyle ayant 1 à 8 atomes de carbone, les groupes alcoxy ayant 1 à 8 atomes de carbone, les groupes alkylthio ayant 1 à 8 atomes de carbone, les groupes alkylamino ayant 1 à 8 atomes de carbone, les groupes dialkylamino ayant 2 à 8 atomes de carbone et les groupes esters d'acides carboxyliques ayant 2 à 8 atomes de carbone ;
où les substituants du type R¹, R², R³ peuvent être identiques ou différents,
et
(B) est au moins un composé cyclopentadiène.

2. Procédé selon la revendication 1, **caractérisé en ce que** R¹ et R², dans le composé de structure (I), sont les groupes tert-butyle, et
R³ = -CN, -COOH, -O-R¹⁰, -S-R¹¹, -R¹² ou -C≡C-R¹³ ;
où
R¹⁰ est le groupe méthyle, éthyle, iso-propyle, n-propyle, n-butyle, iso-butyle, sec-butyle, tert-butyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-timéthylpropyle, 1-éthyl-1-méthylpropyle ou 1-éthyl-2-méthylpropyle ;
R¹¹ est un groupe alkyle ayant 1 à 6 atomes de carbone ; R¹² est le groupe 2-furyle, le groupe 3-furyle ou phényle, où le groupe phényle peut être substitué par au moins un substituant choisi dans le groupe consistant en le groupe hydroxy, les groupes alkyle ayant 1 à 8 atomes de carbone ;
R¹³ est le groupe phényle.

3. Procédé selon la revendication 1 ou 2, **caractérisé en ce que** R¹ et R², dans le composé de formule (I), sont des groupes tert-butyle, et
R³ = -O-R¹⁰ où R¹⁰ est le groupe méthyle, éthyle, n-propyle, iso-propyle, n-butyle, n-pentyle ou n-hexyle.

4. Procédé selon la revendication 1 ou 2, **caractérisé en ce que** R¹ et R², dans le composé de structure (I), sont des groupes tert-butyle, et
R³ = -R¹², où R¹² est le groupe 2-furyle, le groupe 3-furyle ou phényle, de préférence le groupe 2-furyle.

5. Procédé selon l'une ou plusieurs des revendications 1 à 4, **caractérisé en ce que** le composé cyclopentadiène est choisi dans le groupe consistant en le cyclopentadiène, le dicyclopentadiène, le cyclopentadiène alkylé et le dicyclopentadiène alkylé.

6. Procédé selon l'une des revendications 1 à 5, **caractérisé en ce que** le composé cyclopentadiène est choisi dans le groupe consistant en le cyclopentadiène et le dicyclopentadiène.

7. Procédé selon l'une ou plusieurs des revendications 1 à 6, **caractérisé en ce que** (A) se présente sous forme d'une solution (AC) dans (C), où (C) est au moins un solvant, le solvant étant de préférence choisi dans le groupe consistant en le benzène, les composés aromatiques une ou plusieurs fois alkylés, les alcanes ayant un nombre d'atomes de carbone de 6 à 15, les cycloalcanes ayant un nombre d'atomes de carbone de 6 à 15, les fractions hydrocarbonées à haut point d'ébullition, les éthers ayant un nombre d'atomes de carbone de 6 à 15 et les esters ayant un nombre d'atomes de carbone de 6 à 15.

8. Procédé selon la revendication 7, **caractérisé en ce que** le rapport (m/m) entre le poids total de tous les composés de structure (I) dans la solution (AC) et le poids total de tous les solvants dans la solution (AC) est compris dans la plage de 1:1 000 à 100:1.

9. Procédé selon l'une ou plusieurs des revendications 1 à 8, **caractérisé en ce que** la concentration totale de tous les composés de structure (I) est comprise entre 10 ppb (m/m) et 100 000 ppm (m/m), par rapport au poids total de tous les composés cyclopentadiènes.

10. Procédé selon l'une ou plusieurs des revendications 1 à 9, **caractérisé en ce que** (B) se présente dans un courant de procédé à une concentration de 0,0001 à 15 % en poids.

11. Composition (AB) comprenant (A) et (B) dans laquelle
(A) est au moins un composé de structure (I) 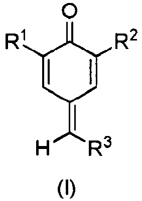 dans laquelle
R¹ et R² représentant chacun indépendamment de l'autre un atome d'hydrogène, un groupe alkyle ayant 1 à 18 atomes de carbone, un groupe cycloalkyle ayant 3 à 15 atomes de carbone, un groupe aryle ayant 6 à 15 atomes de carbone ou un groupe phénylalkyle ayant 7 à 15 atomes de carbone ;
R³ = -CN, -COOH, -COOR⁴, -COR⁵, -OCOR⁶, -CONR⁷R⁸, -PO(OR⁹)₂, -O-R¹⁰, -S-R¹¹, -R¹², -C≡C-R¹³ ou un groupe halogéno ;
où
R⁴, R⁵, R⁶ représentent chacun un groupe alkyle ayant 1 à 18 atomes de carbone, un groupe cycloalkyle ayant 3 à 12 atomes de carbone, un groupe aryle ayant 6 à 10 atomes de carbone ;
R⁷ et R⁸ représentent chacun indépendamment de l'autre : un atome d'hydrogène ;
un groupe alkyle ayant 1 à 15 atomes de carbone, qui peut être substitué par au moins un substituant choisi dans le groupe consistant en les groupes alkylamino ayant 1 à 4 atomes de carbone, les groupes dialkylamino ayant 2 à 8 atomes de carbone, le groupe hydroxy ;
un groupe phénylalkyle ayant 7 à 15 atomes de carbone, qui peut être substitué par au moins un substituant choisi dans le groupe consistant en le groupe hydroxy, les groupes alkyle ayant 1 à 4 atomes de carbone, les groupes alkylamino ayant 1 à 4 atomes de carbone, les groupes dialkylamino ayant 2 à 8 atomes de carbone ;
un groupe aryle ayant 6 à 10 atomes de carbone, qui peut être substitué par au moins un substituant choisi dans le groupe consistant en les groupes alkyle ayant 1 à 4 atomes de carbone, les groupes alkylamino ayant 1 à 4 atomes de carbone, les groupes dialkylamino ayant 2 à 8 atomes de carbone, le groupe hydroxy ;
ou NR⁷R⁸ est le groupe morpholino, pipéridino ou pyrrolidino ;
R⁹, R¹⁰, R¹¹ représentent chacun
un atome d'hydrogène ;
un groupe alkyle ayant 1 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10 et y = 1, 2, 3 ou 4, et R¹⁴ est un groupe alkyle ayant 1 à 6 atomes de carbone ;
un groupe cycloalkyle ayant 3 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10 et y = 1, 2, 3 ou 4, et R¹⁴ est un groupe alkyle ayant 1 à 6 atomes de carbone ;
un groupe phénylalkyle ayant 7 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10, et y = 1, 2, 3 ou 4, et où R¹⁴ est un groupe alkyle ayant 1 à 6 atomes de carbone ;
ou
un groupe aryle ayant 6 à 15 atomes de carbone, qui peut être substitué par un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10, et y = 1, 2, 3 ou 4, et où R¹⁴ est un groupe alkyle ayant 1 à 6 atomes de carbone ;
R¹² est le groupe 2-pyridyle, le groupe 3-pyridyle, le groupe 4-pyridyle, le groupe 2-thiényle, le groupe 3-thiényle, le groupe 2-pyrryle, le groupe 3-pyrryle, le groupe 2-furyle, le groupe 3-furyle ou un groupe aryle ayant 6 à 15 atomes de carbone ; où les radicaux groupe 2-pyridyle, groupe 3-pyridyle, groupe 4-pyridyle, groupe 2-thiényle, groupe 3-thiényle, groupe 2-pyrryle, groupe 3-pyrryle, groupe 2-furyle, groupe 3-furyle ou groupes aryle ayant 6 à 15 atomes de carbone peuvent être substitués par au moins un substituant choisi dans le groupe consistant en le groupe hydroxy, le groupe nitro, le groupe amino, le groupe cyano, le groupe carboxy, le groupe aminocarbonyle, les groupes halogéno, les groupes alkyle ayant 1 à 8 atomes de carbone, les groupes alcoxy ayant 1 à 8 atomes de carbone, les groupes alkylthio ayant 1 à 8 atomes de carbone, les groupes alkylamino ayant 1 à 8 atomes de carbone, les groupes dialkylamino ayant 2 à 8 atomes de carbone et les groupes esters d'acides carboxyliques ayant 2 à 8 atomes de carbone ;
R¹³ est un atome d'hydrogène, un groupe alkyle ayant 1 à 12 atomes de carbone, un groupe aryle ayant 6 à 10 atomes de carbone, où le groupe aryle ayant 6 à 10 atomes de carbone peut être substitué par au moins un substituant choisi dans le groupe consistant en les groupes hydroxy, les groupes nitro, les groupes amino, les groupes cyano, les groupes carboxy, les groupes aminocarbonyle, les groupes halogéno, les groupes alkyle ayant 1 à 8 atomes de carbone, les groupes alcoxy ayant 1 à 8 atomes de carbone, les groupes alkylthio ayant 1 à 8 atomes de carbone, les groupes alkylamino ayant 1 à 8 atomes de carbone, les groupes dialkylamino ayant 2 à 8 atomes de carbone et les groupes esters d'acides carboxyliques ayant 2 à 8 atomes de carbone ;
où les substituants du type R¹, R², R³ peuvent être identiques ou différents,
et
(B) est au moins un composé cyclopentadiène.

12. Composition (AB) selon la revendication 11, **caractérisée en ce que** R¹ et R², dans le composé de structure (I), sont les groupes tert-butyle, et
R³ = -CN, -COOH, -O-R¹⁰, -S-R¹¹, -R¹² ou -C≡C-R¹³ ;
où
R¹⁰ est le groupe méthyle, éthyle, iso-propyle, n-propyle, n-butyle, iso-butyle, sec-butyle, tert-butyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-timéthylpropyle, 1-éthyl-1-méthylpropyle ou 1-éthyl-2-méthylpropyle ;
R¹¹ est un groupe alkyle ayant 1 à 6 atomes de carbone ; R¹² est le groupe 2-furyle, le groupe 3-furyle ou phényle, où le groupe phényle peut être substitué par au moins un substituant choisi dans le groupe consistant en le groupe hydroxy, les groupes alkyle ayant 1 à 8 atomes de carbone ;
R¹³ est le groupe phényle.

13. Composition (AB) selon la revendication 11 ou 12, **caractérisée en ce que** R¹ et R², dans le composé de formule (I), sont des groupes tert-butyle, et
R³ = -O-R¹⁰ où R¹⁰ est le groupe méthyle, éthyle, n-propyle, iso-propyle, n-butyle, n-pentyle ou n-hexyle.

14. Composition (AB) selon la revendication 11 ou 12, **caractérisée en ce que** R¹ et R², dans le composé de structure (I), sont des groupes tert-butyle, et
R³ = -R¹², où R¹² est le groupe 2-furyle, le groupe 3-furyle ou phényle, de préférence le groupe 2-furyle.

15. Composition (AB) selon l'une ou plusieurs des revendications 11 à 14, **caractérisée en ce que** le composé cyclopentadiène est choisi dans le groupe consistant en le cyclopentadiène, le dicyclopentadiène, le cyclopentadiène alkylé et le dicyclopentadiène alkylé.

16. Composition (AB) selon l'une ou plusieurs des revendications 11 à 15, **caractérisée en ce que** le composé cyclopentadiène est choisi dans le groupe consistant en le cyclopentadiène et le dicyclopentadiène.

17. Composition (AB) selon l'une ou plusieurs des revendications 11 à 16, **caractérisée en ce que** la concentration totale de tous les composés de structure (I) dans la composition (AB) est comprise entre 10 ppb (m/m) et 100 000 ppm (m/m), par rapport au poids total de tous les composés cyclopentadiènes dans la composition (AB).

18. Utilisation de (A) pour inhiber la polymérisation de (B),
où
(A) est au moins un composé de structure (I) 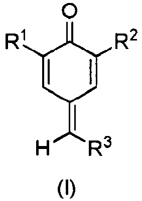 dans laquelle
R¹ et R² représentant chacun indépendamment de l'autre un atome d'hydrogène, un groupe alkyle ayant 1 à 18 atomes de carbone, un groupe cycloalkyle ayant 3 à 15 atomes de carbone, un groupe aryle ayant 6 à 15 atomes de carbone ou un groupe phénylalkyle ayant 7 à 15 atomes de carbone ;
R³ = -CN, -COOH, -COOR⁴, -COR⁵, -OCOR⁶, -CONR⁷R⁸, -PO(OR⁹)₂, -O-R¹⁰, -S-R¹¹, -R¹², -C≡C-R¹³ ou un groupe halogéno ;
où
R⁴, R⁵, R⁶ représentent chacun un groupe alkyle ayant 1 à 18 atomes de carbone, un groupe cycloalkyle ayant 3 à 12 atomes de carbone, un groupe aryle ayant 6 à 10 atomes de carbone ;
R⁷ et R⁸ représentent chacun indépendamment de l'autre : un atome d'hydrogène ;
un groupe alkyle ayant 1 à 15 atomes de carbone, qui peut être substitué par au moins un substituant choisi dans le groupe consistant en les groupes alkylamino ayant 1 à 4 atomes de carbone, les groupes dialkylamino ayant 2 à 8 atomes de carbone, le groupe hydroxy ;
un groupe phénylalkyle ayant 7 à 15 atomes de carbone, qui peut être substitué par au moins un substituant choisi dans le groupe consistant en le groupe hydroxy, les groupes alkyle ayant 1 à 4 atomes de carbone, les groupes alkylamino ayant 1 à 4 atomes de carbone, les groupes dialkylamino ayant 2 à 8 atomes de carbone ;
un groupe aryle ayant 6 à 10 atomes de carbone, qui peut être substitué par au moins un substituant choisi dans le groupe consistant en les groupes alkyle ayant 1 à 4 atomes de carbone, les groupes alkylamino ayant 1 à 4 atomes de carbone, les groupes dialkylamino ayant 2 à 8 atomes de carbone, le groupe hydroxy ;
ou NR⁷R⁸ est le groupe morpholino, pipéridino ou pyrrolidino ;
R⁹, R¹⁰, R¹¹ représentent chacun
un atome d'hydrogène ;
un groupe alkyle ayant 1 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10 et y = 1, 2, 3 ou 4, et R¹⁴ est un groupe alkyle ayant 1 à 6 atomes de carbone ;
un groupe cycloalkyle ayant 3 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10 et y = 1, 2, 3 ou 4, et R¹⁴ est un groupe alkyle ayant 1 à 6 atomes de carbone ;
un groupe phénylalkyle ayant 7 à 15 atomes de carbone, qui peut être substitué par au moins un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10, et y = 1, 2, 3 ou 4, et où R¹⁴ est un groupe alkyle ayant 1 à 6 atomes de carbone ;
ou
un groupe aryle ayant 6 à 15 atomes de carbone, qui peut être substitué par un substituant choisi dans le groupe consistant en le groupe hydroxy, les groupes dialkylamino, -OR¹⁴, -[O(CH₂)_{y}]ₓH, où x = 1, 2, 3, 4, 5, 6, 7, 8, 9 ou 10, et y = 1, 2, 3 ou 4, et où R¹⁴ est un groupe alkyle ayant 1 à 6 atomes de carbone ;
R¹² est le groupe 2-pyridyle, le groupe 3-pyridyle, le groupe 4-pyridyle, le groupe 2-thiényle, le groupe 3-thiényle, le groupe 2-pyrryle, le groupe 3-pyrryle, le groupe 2-furyle, le groupe 3-furyle ou un groupe aryle ayant 6 à 15 atomes de carbone ; où les radicaux groupe 2-pyridyle, groupe 3-pyridyle, groupe 4-pyridyle, groupe 2-thiényle, groupe 3-thiényle, groupe 2-pyrryle, groupe 3-pyrryle, groupe 2-furyle, groupe 3-furyle ou groupes aryle ayant 6 à 15 atomes de carbone peuvent être substitués par au moins un substituant choisi dans le groupe consistant en le groupe hydroxy, le groupe nitro, le groupe amino, le groupe cyano, le groupe carboxy, le groupe aminocarbonyle, les groupes halogéno, les groupes alkyle ayant 1 à 8 atomes de carbone, les groupes alcoxy ayant 1 à 8 atomes de carbone, les groupes alkylthio ayant 1 à 8 atomes de carbone, les groupes alkylamino ayant 1 à 8 atomes de carbone, les groupes dialkylamino ayant 2 à 8 atomes de carbone et les groupes esters d'acides carboxyliques ayant 2 à 8 atomes de carbone ;
R¹³ est un atome d'hydrogène, un groupe alkyle ayant 1 à 12 atomes de carbone, un groupe aryle ayant 6 à 10 atomes de carbone, où le groupe aryle ayant 6 à 10 atomes de carbone peut être substitué par au moins un substituant choisi dans le groupe consistant en les groupes hydroxy, les groupes nitro, les groupes amino,
les groupes cyano, les groupes carboxy, les groupes aminocarbonyle, les groupes halogéno, les groupes alkyle ayant 1 à 8 atomes de carbone, les groupes alcoxy ayant 1 à 8 atomes de carbone, les groupes alkylthio ayant 1 à 8 atomes de carbone, les groupes alkylamino ayant 1 à 8 atomes de carbone, les groupes dialkylamino ayant 2 à 8 atomes de carbone et les groupes esters d'acides carboxyliques ayant 2 à 8 atomes de carbone ;
où les substituants du type R¹, R², R³ peuvent être identiques ou différents,
et
(B) est au moins un composé cyclopentadiène.

19. Utilisation selon la revendication 18, **caractérisée en ce que** R¹ et R², dans le composé de structure (I), sont les groupes tert-butyle, et
R³ = -CN, -COOH, -O-R¹⁰, -S-R¹¹, -R¹² ou -C≡C-R¹³ ;
où
R¹⁰ est le groupe méthyle, éthyle, iso-propyle, n-propyle, n-butyle, iso-butyle, sec-butyle, tert-butyle, n-pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, n-hexyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-timéthylpropyle, 1-éthyl-1-méthylpropyle ou 1-éthyl-2-méthylpropyle ;
R¹¹ est un groupe alkyle ayant 1 à 6 atomes de carbone ;
R¹² est le groupe 2-furyle, le groupe 3-furyle ou phényle, où le groupe phényle peut être substitué par au moins un substituant choisi dans le groupe consistant en le groupe hydroxy, les groupes alkyle ayant 1 à 8 atomes de carbone ;
R¹³ est le groupe phényle.

20. Utilisation selon la revendication 18 ou 19, **caractérisée en ce que** R¹ et R², dans le composé de formule (I), sont des groupes tert-butyle, et
R³ = -O-R¹⁰ où R¹⁰ est le groupe méthyle, éthyle, n-propyle, iso-propyle, n-butyle, n-pentyle ou n-hexyle.

21. Utilisation selon la revendication 18 ou 19, **caractérisée en ce que** R¹ et R², dans le composé de structure (I), sont des groupes tert-butyle, et
R³ = -R¹², où R¹² est le groupe 2-furyle, le groupe 3-furyle ou phényle, de préférence le groupe 2-furyle.

22. Utilisation selon l'une ou plusieurs des revendications 18 à 21, **caractérisée en ce que** le composé cyclopentadiène est choisi dans le groupe consistant en le cyclopentadiène, le dicyclopentadiène, le cyclopentadiène alkylé et le dicyclopentadiène alkylé.

23. Utilisation selon l'une ou plusieurs des revendications 18 à 22, **caractérisée en ce que** le composé cyclopentadiène est choisi dans le groupe consistant en le cyclopentadiène et le dicyclopentadiène.
